(19) Europäisches Patentamt
European Patent Office
Office européen des brevets

(11) EP 1 858 503 B1

(12) **EUROPEAN PATENT SPECIFICATION**

(45) Date of publication and mention of the grant of the patent:
**04.09.2013 Bulletin 2013/36**

(21) Application number: **06736159.2**

(22) Date of filing: **27.02.2006**

(51) Int Cl.:
***A61K 31/192*** (2006.01) ***A61K 9/06*** (2006.01)
***A61K 47/32*** (2006.01) ***A61P 29/00*** (2006.01)
***A61P 17/00*** (2006.01) ***A61P 17/06*** (2006.01)

(86) International application number:
**PCT/US2006/006780**

(87) International publication number:
**WO 2006/096360 (14.09.2006 Gazette 2006/37)**

(54) **TOPICAL GELS COMPOSITIONS**

TOPISCHE GELZUSAMMENSETZUNGEN

COMPOSITIONS DE GELS TOPIQUES

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.03.2005 US 658084 P**
**13.05.2005 US 681102 P**
**14.06.2005 US 690201 P**
**24.02.2006 US 361384**

(43) Date of publication of application:
**28.11.2007 Bulletin 2007/48**

(73) Proprietor: **Green, Monique Renata**
**East St. Louis, IL 62205 (US)**

(72) Inventors:
- **SPANN-WADE, Monique**
**Chesterfield, MO 63005-5405 (US)**
- **WARD, Anthony, J.**
**St. Louis, MO 63131 (US)**

(74) Representative: **Samson & Partner**
**Widenmayerstrasse 5**
**80538 München (DE)**

(56) References cited:
**EP-A- 0 439 344** **WO-A-87/02891**
**US-A- 5 914 322** **US-A- 5 976 566**

- **BANSAL A K ET AL: "Alkyl ester prodrugs for improved topical delivery of ibuprofen." INDIAN JOURNAL OF EXPERIMENTAL BIOLOGY. MAR 2001, vol. 39, no. 3, March 2001 (2001-03), pages 280-283, XP009067459 ISSN: 0019-5189**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 858 503 B1

## Description

CROSS-REFERENCE TO RELATED APPLICATION

This application claims priority to U.S. Provisional Patent Application No. 60/658,084, filed March 3, 2005; U.S. Provisional Patent Application No. 60/681,102, filed May 13, 2005; and U.S. Provisional Patent Application No. 60/690,201, filed June 14, 2005.

TECHNICAL FIELD

The invention relates to compositions which may be used for treating pain resulting from local stimulation of nociceptors in skin, bones, joints, and muscles and in skin disorders wherein inflammation is a component of the pathogenesis. An example of such an inflammatory skin disorder is pseudofolliculitis barbae.

FIELD OF THE INVENTION

The pathogenesis of a wide variety of skin disorders involves an inflammatory process. Often, such disorders involve inflammatory cells (e.g., polymorphonuclear neutrophils and lymphocytes) infiltrating the skin with no overt or known infectious etiology. Symptoms of inflammatory skin conditions generally include erythema (redness), edema (swelling), pain, pruritus, increased surface temperature and loss of function.

While a range of treatments have been developed for inflammatory skin conditions, none are completely effective or free of adverse side effects. Treatments for different inflammatory skin conditions typically include topical or oral steroids (e.g., for various types of eczema, acne, and erythema multiforme); ultraviolet light (e.g., for nummular eczema and mycosis fungoides); antibiotics, and other anti-inflammatory therapies.

Corticosteroids have the greatest importance for the treatment of inflammatory skin disorders. Weak to medium strong corticosteroids (e.g., nonfluorinated derivatives of hydrocortisone) are mainly employed for the therapy of inflammatory, allergic and pruritic skin disorders. While short-term treatment (a few days or weeks) with oral steroids is relatively safe, long-term treatment (more than 3 months) may cause undesirable side effects including Cushing's syndrome, skin thinning, and increased susceptibility to infection. In addition, improvements may be delayed, such as with the various acne treatments, lasting several months.

There are also a variety of agents commonly used in medical practice which are nonnarcotic and nonsteroidal, but which nevertheless can be used to combat both inflammation and pain. These are the salicylates and also agents which are often termed nonsteroidal anti-inflammatory drugs (NSAIDs).

There are now a variety of newer drugs available. Although the chemical structures of these newer agents vary quite widely, a common structural feature of many of these compounds is the presence of a carboxylic acid group (COOH). For example, one group of NSAIDs consists of propionic acid derivatives (the so-called "profens," e.g., ibuprofen), and another group of NSAIDs consists of acetic acid derivatives (e.g., indomethacin).

NSAIDs can cause gastric ulcers and bleeding on long-term oral use. A goal of topical administration of NSAIDs is to deliver therapeutically effective levels of drug to the local target (e.g., nociceptors and inflammatory cells in the skin) while bypassing the stomach and preventing systemic delivery.

Unfortunately, NSAIDs are often not well absorbed when administered topically. Those topical formulations that do provide some absorption through the skin can result in substantial systemic delivery and often fail to provide therapeutic levels in the skin.

In addition, acute inflammation and pain are often treated by the topical administration of a counterirritant. In this regard, a widely used agent is methyl salicylate, which is often applied to the skin in the form of an ointment or cream and which elicits a soothing, mildly analgesic effect. However, methyl salicylate suffers from the disadvantage that it possesses an odor, which under certain circumstances, and to certain individuals, can be regarded as unpleasant.

U.S. Patent 4,185,100 entitled, "Topical Anti-Inflammatory Drug Therapy," describes a method of topical treatment of an inflammatory condition of the skin comprising applying to the affected area a nonsteroidal anti-inflammatory agent and concurrently a topically active anti-inflammatory corticosteroid. These agents are applied in a dermatologically-acceptable, topical vehicle selected from the group consisting of creams, gels, ointments, powders, aerosols and solutions suitable for topical administration.

Kyuki et al., "Anti-Inflammatory Effect of Diclofenac-Sodium Ointment (Cream) in Topical Application," Japan J. Pharmacol. 33, 121-132 (1983), describes the anti-inflammatory effect of a diclofenac-sodium. Ointments were prepared with three kinds of bases: lithophilic, emulsion (cream) and gel bases and their anti-inflammatory effects were compared. The cream base was reported by Kyaki et al. to have the most potent effect.

EP Published Patent Application EP 0151953, entitled "Topical Drug Release System," describes on pages 10-11 an ibuprofen CARBOPOL® gel system containing ibuprofen, propylene glycol, water, CARBOPOL® 940 (polyacrylic

acid polymer) and diisopropanolamine, as an illustrative example of a pharmaceutical composition for percutaneous absorption by topical application made in two liquid drug-containing phases, which are to be mixed together *in situ* just before use to form a supersaturated drug-containing gel. The EPO application discloses a nonalcoholic gel system for delivering ibuprofen topically.

U.S. Patent 5,093,133, entitled "Method for Percutaneous Delivery of Ibuprofen Using Hydroalcoholic Gel," describes a hydroalcoholic gel comprising ibuprofen, a hydroxypropylcellulose or polyacrylic acid polymer, with propylene glycol being an optional but preferred ingredient. The patent further teaches the desirability of adding alkalinizing agent to the formulation to increase percutaneous absorption, the desirability of water, and the use of the S-enantiomer.

U.S. Patent 4,533,546, entitled "Anti-Inflammatory Analgesic Gelled Ointments," Kishi et al., discloses NSAID-containing (e.g., ibuprofen) hydroalcoholic gels having a pH in the range of 7.0 to 9.0. The gel ointment comprises a phenylacetic acid anti-inflammatory compound, a carboxyvinyl polymer, a water-soluble organic amine (e.g., triethanolamine), and water wherein the amount of organic amine is such that the gel ointment has a pH in the range of 7.0 to 9.0, and preferably 7.3 to 7.8.

Topical gels containing ibuprofen have been described in U.S. Patent 6,277,362, Ita, issued August 21, 2001, for treatment of pseudofolliculitis barbae (PFB). Pseudofolliculitis barbae is a skin disorder primarily affecting subjects who shave curly hairs. A coiled hair tends to grow by curving backward toward the skin. Over the course of a single day's growth, the tip of the hair shaft may press back into the skin. Since the razor leaves a sharp sheared edge on the hair tip, the hair may actually penetrate the skin and continue proceeding inward.

The epidermis (i.e., the outermost layer of the skin) contains keratinocytes. In response to penetration (e.g., by a hair), keratinocytes and other nonhematopoieticallyderived resident cells produce various cytokines which stimulate migration of T cells and expression of adhesion molecules. As a result, inflammatory cells (e.g., polymorphonuclear neutrophils and lymphocytes) infiltrate the skin (from the dermis), resulting in a swollen bump in the region.

Full-blown PFB is typically characterized by irritating bumps, itchiness, and discoloration of the affected areas. PFB becomes part of an accelerating cycle. The bumps are present the next time shaving takes place, resulting in a cut of the raised area and further irritation. Additionally, complications of PFB include cellulitis, furunculosis, and hypertrophic or keloid scars. Secondary bacterial infection can also result from PFB.

Prior art known to the inventors concerning the subject of PFB includes the following references: U.S. Patent 3,981,681, issued to Mario de la Guarida, on Sep. 21, 1976; U.S. Patent 4,228,163, issued to William E. Bliss, on Oct. 14, 1980; U.S. Patent 4,525,344, issued to Ronald J. Tutsky, on Jun. 25, 1985; U.S. Patent 4,775,530, issued to Nicholas V. Perricone, on Oct. 4, 1988; and U.S. Patent 5,034,221, issued to Steven E. Rosen et al., on Jul. 23, 1991.

Typically, topical formulations and particularly gel formulations are thickened using well-known polymeric thickeners, such as the CARBOPOL® materials which are copolymers or polymers of polyacrylic acids. Conventional use of such polymers as thickeners in topical formulations requires that the polymers be neutralized in order to get the appropriate thickening performance. Thus, for example, Fresno, et al., Eur. J. Pharm. Biopharm.:54:329-335 (2002), states the following: "Like in the case of other CARBOPOL™ resins, neutralization of ULTREZ™ 10 dispersions is essential to develop the rheological, and consequently, the mechanical properties of the polymer...." Topical formulations which require the use of neutralized polymeric thickeners are also disclosed in U.S. Patent 5,976,566, Samour, et al., issued November 2, 1999, and Akbari, et al., FIP World Congress Proceedings, Nice, France (2002).

US 5,914,322 A discloses NSAID formulations comprising hyaluronic acid. EP 0 439 344 A2 describes ibuprofen containing hydroalcoholic gels comprising ibuprofen. Keratolytic agents are not mentioned. US 5,976,566 A discloses topical alcoholic and aqueous alcoholic gels comprising ibuprofen or other NSAIDs and skin penetration enhancers. WO 87/02891 discloses the reaction product of a NSAID and methyl salicylate, namely a NSAID-methyl salicylate ester, for topical administration. A.K. Bansal et al., Indian Journal of Experimental Biology (March 2001), vol. 39, No. 3, pp. 280-283, discloses alkyl ester drugs for improved topical delivery of ibuprofen.

What is needed in the art is a topical formulation that provides delivery of effective concentrations of an active drug to treat an inflammatory skin condition with favorable rheologic properties, minimal systemic delivery, and rapid epidermal and dermal delivery.

## SUMMARY OF THE INVENTION

New compositions have been discovered that, when topically applied, deliver therapeutic levels of an NSAID to an individual with a local inflammatory disorder.

Surprisingly, it has been discovered that compositions of the present invention have one or more advantageous pharmacodynamic properties and provide therapeutic levels of NSAID for a diverse range of local inflammatory disorders. Moreover, therapeutic levels of an NSAID are attained with minimal systemic delivery.

Described herein is a composition comprising an NSAID prodrug, a solvent, and a thickening agent wherein the NSAID prodrug is a phenylacetic acid-type NSAID unsubstituted alkyl ester wherein the thickening agent is optionally a polymeric thickening agent.

A composition comprising a composition comprising an NSAID, a solvent, and at least one excipient selected from thickeners, humectants, keratolytics, oils, emollients, surfactants, preservatives, colorants, UV blockers, antioxidants, and perfumes is also disclosed.

Further described is a method of treating an inflammatory skin disorder comprising topically administering to a subject in need thereof, an NSAID prodrug , wherein the NSAID prodrug is a phenylacetic acid-type NSAID alkyl ester and wherein the subject is a human, a livestock animal (e.g., beef and dairy cattle, sheep, poultry, and swine, etc.), or a companion animal (dogs, cats, horses, etc).

An alcoholic gel composition described herein may comprise: one or more alcoholic solvents in an amount of about 10% to about 90%, one or more NSAIDs in a total amount of about 0.001% to about 25%, a polymeric thickener in an amount of about 0.05% to about 5%, and one or more keratolytic agents are present in a total keratolytic agent concentration amount of about 0.015% to about 25, and wherein the NSAID is substantially dissolved in the one or more alcoholic solvents.

One composition described herein comprises: one or more alcoholic solvents in an amount of about 50% to about 70%, an NSAID in a total amount of about 5% to no more than about 25%, and a polymeric thickener in an amount of about 0.05% to about 2%, and water in an amount from 0 to about 20%.

The composition according to the invention comprises: one or more alcoholic solvents in an amount of about 10% to about 90%, one or more NSAID selected from ibuprofen salt, ibuprofen free acid and esters thereof in a total amount of about 0.001% to about 25%, one or more keratolytic agents including $\alpha$- and $\beta$-hydroxylic acids and $\beta$-ketocarboxylic acids in a total of .015% to 25% and a polymeric thickener in an amount of about 0.05% to about 5%, wherein the composition has a viscosity of about 2,000 cps to about 200,000 cps without the addition of an alkalinizing agent.

In one embodiment, an alcoholic gel composition comprises at least one alcoholic solvent present in a total amount from about 30% to about 90%, at least one NSAID having a carboxylic acid group, and at least one polymeric thickener selected from the group consisting of polyacrylic acid thickeners and alkylhydroxycellulose thickeners present in a total thickener amount of about 0.1% to about 5%, wherein upon storage of the composition, ester formation between the at least one alcoholic solvent and the carboxylic acid group is less than about 0.03% per day.

Also provided in another embodiment is a method of treating a local inflammatory disorder comprising applying to the skin of a subject in need thereof any topically acceptable composition of the present invention.

Also provided in another embodiment is delivery systems (including a storage devices) useful for delivering any of the compositions of the present invention.

Optionally the inflammatory skin disorder is pseudofolliculitis barbae.

## BRIEF DESCRIPTION OF THE DRAWINGS

Figure 1 shows the viscosity-stabilizing effects of salicylic acid with storage.

Figure 2 shows the pH-stabilizing effects of salicylic acid with storage.

Figure 3 shows the stabilizing effects of salicylic acid with storage as a pH vs. viscosity plot.

Figure 4 shows plots of log10P vs. viscosity change upon addition of various active drugs.

Figure 5 shows percutaneous absorption of present compositions.

Figure 6 shows the UV chromatogram (220 nm) of HPLC following injection of Formula 1a stored 3 months ~ 25°C.

Figure 7a shows the positive ESI mass spectrum for the ibuprofen peak.

Figure 7b shows the UV spectrum for the ibuprofen.

Figure 8a shows the positive ESI mass spectrum obtained from the prodrug.

Figure 8b shows the UV spectrum obtained from the prodrug

Figure 9 shows prodrug generation with time of storage and the positive effect of salicylic acid.

Figure 10 shows prodrug generation with time of storage and the negative effect of alkalinizing agent addition.

Figure 11 shows prodrug generation with time of storage and the negative effect of alkalinizing agent addition - longer study.

Figure 12 shows prodrug generation with time of storage and the effect of alkalinizing agent addition and NSAID concentration.

Figure 13 shows the effect of the keratolytic agent salicylic acid on prodrug formation upon storage of composition 1a.

## DETAILED DESCRIPTION OF THE INVENTION

As used herein, the following definitions apply:

Definitions

"%", in reference to a concentration of a component of a composition, means the ratio of weight of a component to total weight expressed as a percent, unless otherwise stated.

"Solvent" means a solvent or solvent system, wherein, a substantial portion of a topically active drug may be solubilized therein, in compositions of the present invention.

"Alkalinizing agent" means an agent known in the art to be usefully added to a composition in order to increase the pH of the composition. Nonlimiting examples of such alkalinizing agents include ammonium hydroxide, alkaline earth metal salts such as magnesium oxide, magnesium hydroxide, calcium hydroxide, sodium hydroxide, potassium hydroxide, lithium hydroxide, aluminum hydroxide, potassium carbonate, sodium bicarbonate, and the like. Other examples include organic basic salts such as alkanolamines such as methanolamine, ethanolamine, propanolamine, butanolamine, dimethanolamine diethanolamine, dipropanolamine, dibutanolamine, diisopropanolamine, trimethanolamine triethanolamine, tripropanolamine, diisopropanolamine, tributanolamine, aminomethylpropanol, N-methyl glucamine, tetrahydroxypropyl ethylene diamine, and the like; alkylamines such as methylamine, ethylamine, propylamine, butylamine, diethylamine, dipropylamine, isopropylamine, and the like.

"Disorder" means any abnormal pathology. A disorder can be inherited, infectious, acquired, induced (e.g., contact dermatitis or inflammation following surgical incision), chronic, or acute.

"Excipients" means any material that is combined with a drug in order to produce a drug dosage form. Nonlimiting examples of excipients include, for example, thickeners, humectants, keratolytics, oils, emollients, surfactants, preservatives, colorants, UV blockers, antioxidants, perfumes, mineral oil, liquid petrolatum, white petrolatum, glycerin, polyethylene glycol and propylene glycol.

"Thickening agent" means any agent useful as an aid to thicken or add structure to a topical formulations. These agents impart physical stability and increased viscosity. Nonlimiting examples of thickening agents are gums and natural polysaccharides, mineral thickeners, oils, and synthetic polymeric thickeners. Additionally, a thickening agent refers to one or more agents that, in combination, result in a viscosity suitable for dermatologic applications.

"Topically active drug" means a pharmaceutical or botanical compound that can be applied to the skin in a useful composition with an activity that has therapeutic efficacy against a local target. Topically active drugs include all drug polymorphs, crystal habits thereof, prodrugs and isomers thereof (including optical, geometric and tautomeric isomers), enantiomers. salts, solvates and complexes thereof and solvates and complexes of salts thereof.

"Local Targets" means, by way of example, skin, joints, muscle, and ligaments.

"Local Inflammatory disorder" means a disorder wherein an inflammatory process is a component of a disorder of a local target.

"Prodrug" means a pharmacologically inactive or less active chemical derivative of a topically active drug can be converted to the more active form ("parent drug") by an enzymatic or chemical hydrolysis *in vivo.* The prodrug consists of the parent drug covalently linked to another compound (the "pro-moiety"). As used herein, prodrug does not include an NSAID derivative formed by esterification at an NSAID carboxylic acid functionality with an acyloxyalkyl radical. "Prodrug ester" denotes a prodrug wherein the pro-moiety is in ester linkage to the parent drug.

"Safe and effective amount" means an amount of the composition which is sufficient to provide adequate treatment of the condition being treated, but is not so great as to provide undesirable side effects to the user.

"Substantially alkalinizing agent-free" means that the composition comprises no alkalinizing agent other than an alkalinizing agent that is present as a contaminant of another component used in preparing such a composition.

"Treatment" means curative, palliative and/or prophylactic treatment. "Treatment" is not meant to indicate a quantitative effect, but rather that there has been a clinically observable beneficial effect. For example, prophylactic treatment includes a situation where a composition of the present invention is administered to a subject before symptoms are observable and symptoms subsequently occur, but to a lesser degree than without administration.

"Topically acceptable" and "dermatologically acceptable" composition means that, when applied to the skin, there is no significant skin irritation under normal usage circumstances with typical patients.

"Viscosity" means liquid fluidity as measured by a Brookfield DV-III Ultra Programmable Rheometer, spindle #LV4, 10 rpm.

Compositions according to the present invention have one or more superior feature desired in a topical formulation, namely (1) pH stability; (2) viscosity stability; (3) minimal systemic delivery; (4) rapid delivery of therapeutic levels of a topically active drug to the skin; (5) delivery of high levels of a topically active drug to the skin; (6) delivery of sustained therapeutic levels of a topically active drug for an extended period of time; (7) rheologic properties that increase skin exposure to the topically active drug (8).

Prodrug Compositions

NSAID prodrugs can be formulated into compositions such that there is superior drug delivery to local targets

yet systemic delivery remains minimal. Without being bound by theory, it is believed that the hydrophobic nature of the NSAID prodrugs allows for superior dermal delivery. Such delivery is followed by release of the pro-moiety by resident enzymes in the skin (e.g., esterases), converting the prodrug to the less hydrophobic, parent drug. This less hydrophobic drug has reduced ability to diffuse further to the more vascularized regions.

A prodrug of a topically active drug has reduced or no pharmacological activity, but when administered topically, the drug is converted into a drug having the desired activity (the parent drug). Exemplary prodrugs include NSAID prodrugs, for example, NSAID prodrugs of the phenylacetic acid type. Other exemplary NSAIDs and NSAID classes are disclosed elsewhere herein. Those skilled in the art will readily recognize a functionality on a topically active drug that is useful for derivitization to add the promoiety through a bond to the NSAID that can be processed in local tissues to form the parent drug.

Selection of the pro-moiety allows for modulation of dipole moment, charge, diffusion rate, and rate of hydrolytic cleavage to form the "parent" drug.

Prodrugs can be formed from a parent drug, for example, by adding a promoiety through esterification of a carboxylic acid functionality (for example, aryl carboxylic acid derivative NSAIDs). The hydrogen of the hydroxyl group of the carboxylic acid is replaced, for example, by alky or aryl or carbonyl. An alkyl can be unsubstituted or substituted, for example, such as alkyloxyalkyl, alkoxycarbonylalkyl, alkoxycarbonylaminoalkyl, aminoalkyl, or alkylcarbonylaminoalkyl.

Other examples of pro-moieties are methyl, ethyl, isopropyl, n-propyl, tert-butyl, butyl, pentyl, methoxy, tert-butoxy, methoxyethyl, ethoxymethyl, methoxymethyl, phenyl, carboxyethyl, methoxycarbonylmethyl, methoxycarbonylethyl, tert-butoxycarbonylaminomethyl, methoxycarbonyl, aminomethyl, and methylcarbonyl-aminomethyl; or a pharmaceutically acceptable salt thereof.

A prodrug can also be produced to form an amide ester or a thioester.

A prodrug can be formed in an NSAID by, for example, adding a pro-moiety to the NSAID through ether formation at a hydroxyl functionality wherein the hydrogen of the hydroxyl functionality is replaced by an alkanoyloxyalkyl.

A pro-moiety can also be linked to an NSAID through formation of carbonates, carbamates, and amides covalently bonded through the carbonyl carbon.

Methods of preparation of prodrugs are described herein. Additional methods are described in, for example U.S. Patent 5,073,641, U.S. Patent 5,998,465, U.S. Patent 5,811,438, U.S. Patent 6,730,696, U.S. Patent 6,620,813, U.S. Patent 6,143,734, U.S. Patent 5,750,564, U.S. Patent 5,484,833, U.S. Patent 5,315,027, U.S. Patent 4,990,658, U.S. Patent 4,851,426, U.S. Patent 4,049,700, and U.S. Patent 3,228,831.

Topically active drugs, are optionally poorly soluble, practically water insoluble, or water insoluble.

Optionally, topically active drugs contain a carboxylic acid functionality and/or a hydroxyl functionality.

Optionally, topically active drugs contain a carboxylic acid functionality and/or a hydroxyl functionality and are water insoluble or practically water insoluble.

## Superior Properties

It is now possible to prepare compositions with different pharmacodynamic properties by selection of the NSAID, pro-moiety, and solvent. Compositions additionally provide one or more superior topical formulation features when compared to the corresponding parent NSAID (e.g., ketoprofen is the corresponding parent NSAID of ketoprofen isobutyl ester): (1) higher levels of drug in the skin or deeper tissue (e.g., joints or muscles); (2) more sustained level of an NSAID in the skin or deeper tissue (e.g., joints or muscles); and/or (3) more rapid delivery of an NSAID in the skin or deeper tissue (e.g., joints or muscles).

Moreover, NSAID prodrug esters can be topically applied in a variety of compositions. Compositions comprising such prodrugs, can generally be made to contain greater amounts of such prodrug when compared to the corresponding parent NSAID.

Compositions comprising NSAID prodrugs are especially useful for conditions where it is desirable to rapidly produce levels of an NSAID at a target site.

Compositions comprising NSAID prodrugs are especially useful for conditions where it is desirable to achieve penetration.

Compositions comprising such prodrugs can have reduced alcohol at a given concentration of prodrug when compared to the corresponding NSAID. Such reduced alcohol compositions are useful for local inflammatory disorders where alcohol is undesirable (e.g., conditions where drying agents are contraindicated). Such undesirable conditions include conditions where it is undesirable to dry or further dry the skin. Examples of such disorders especially useful for treatment with a reduced alcohol compositions of NSAID prodrug esters are psoriasis and dermatitis.

NSAID prodrug compositions can be gels, hydrogels, lotions, solutions, creams, ointments, dusting powders, dressings, foams, films, skin patches, wafers, implants, sponges, fibres, bandages, microemulsions, and/or liposomes. Optional carriers include alcohol, water, mineral oil, liquid petrolatum, white petrolatum, glycerin, polyethylene glycol

and propylene glycol. Penetration enhancers may be incorporated.

NSAID prodrug compositions can be prepared by dissolving all or substantially all of an NSAID prodrug in a solvent. Nonlimiting examples of useful solvents or solvent systems are alcohols, organogels, complexing agents, cyclodextrins, liposomes, microsomes, phospholipids/copolymers, and oil-in-water emulsions. NSAID prodrug compositions can also be prepared without any significant addition of solvent.

Solvents have surprising effect on drug delivery. Without being bound by theory, the inventors believe that NSAIDs are absorbed into the skin by two different mechanisms: diffusion from the solvent and transport concurrently with the solvent. Both mechanisms are competed with by evaporation of the solvent, especially in the case of volatile solvents. However, in high NSAID compositions (e.g., about 5% or greater), NSAID absorption through both mechanisms can be substantially accelerated. This is believed to result in faster drug delivery, high drug levels at target sites, and deeper penetration. Nevertheless, the more hydrophilic nature of the dermis can result in the surprisingly minimal systemic delivery ofNSAIDs in compositions containing an alcohol solvent.

Because NSAID prodrugs generally have increased alcohol solubility when compared to the corresponding NSAIDs, it is possible to prepare a dermatologically acceptable composition with reduced content of an alcohol solvent (or other organic solvent).

Formulations

Herein described is a composition comprising an NSAID prodrug, a solvent, and a thickening agent wherein the NSAID prodrug is a phenylacetic acid-type NSAID unsubstituted alkyl ester wherein the thickening agent is optionally a polymeric thickening agent (such agents described elsewhere herein). Optionally, the thickening agent is present in an amount of about 0.05% to about 5%.

Also described herein a composition comprising an NSAID prodrug, a solvent, and a thickening agent wherein the NSAID prodrug is an unsubstituted alkyl ester of an NSAID other than naproxen wherein the thickening agent is optionally a polymeric thickening agent (such agents described elsewhere herein).

Furthermore, there is a composition described comprising an NSAID prodrug, a solvent, and a thickening agent wherein the NSAID prodrug is of the NSAID type selected from the group consisting of phenyl acetic-type NSAID, mefanamic-type NSAID, oxicam-type NSAID, and indomethacin type NSAID, and wherein the NSAID prodrug is an unsubstituted alkyl ester.

A composition comprising an $C_1$-$C_3$ carbon unsubstituted alkyl ester NSAID prodrug, a solvent, and a thickening agent, is also described.

Described is a composition comprising an ester NSAID prodrug, a solvent, and a thickening agent, wherein the NSAID prodrug is an ibuprofen prodrug, and wherein the promoiety is an ester linkage (i.e., ester-linked) to the NSAID and wherein the promoiety is an amidyl, a thio, and/or an unsubstituted alkyl.

In the present invention, the thickening agent is a polymeric thickening agent (such agents described elsewhere herein). Optionally, the thickening agent is present in an amount of 0.05% to 5%.

Furthermore, there is described a composition comprising an NSAID, an NSAID prodrug, a solvent, and at least one excipient such as a thickener, a humectant, a keratolytic, an oil, an emollient, a surfactant, a preservative, a colorant, a UV blocker, an antioxidant, or a perfume. Optionally, the NSAID prodrug can be metabolized to form the NSAID (e.g., coformulation of flurbiprofen and flurbiprofen ethyl ester).

Compositions comprising an NSAID and a NSAID prodrug have surprisingly beneficial effects on local inflammatory disorders. Without being bound by theory, it is believed that the NSAID prodrug results in more rapid diffusion and greater localization than the corresponding parent NSAID. The prodrug, after being delivered to the target tissue, is converted to the parent NSAID. It is believed that the 100% conversion to the parent NSAID is not instantaneous upon absorption into the skin. It believed that the NSAID prodrug is not as active as the parent drug at the site of action. The NSAID in the composition generally provides a slower drug delivery as a result of the NSAID's lower hydrophobicity but provides for higher activity once at a local site. Regardless of the mechanism, the NSAID prodrug/NSAID combination results in compositions with not only rapid and sustained delivery, but higher local concentration of active drug to target tissues.

The coformulation of an NSAID and NSAID prodrug can be manufactured by a step of combining an NSAID, an NSAID prodrug, a solvent, and optionally one or more excipients to form a dermatologically acceptable composition.

Optionally, the solvent in an NSAID prodrug composition can be an alcoholic solvent or a nonalcoholic solvent.

Herein described is a method of treating a epidermal inflammatory disorder comprising topically administering to a subject in need thereof, an ibuprofen prodrug, wherein the epidermal inflammatory is selected from the group consisting of psoriasis, folliculitis, PFB, and/or dermatitis. Dermatitis can, for example, be contact dermatitis, occupationally acquired dermatitis, and the like.

The aforementioned NSAID prodrug compositions can be gels, hydrogels, lotions, solutions, creams, ointments, dusting powders, dressings, foams, films, skin patches, wafers, implants, sponges, fibres, bandages, microemulsions,

and/or liposomes. Optional carriers include alcohol, water, mineral oil, liquid petrolatum, white petrolatum, glycerin, polyethylene glycol and propylene glycol. Penetration enhancers may be incorporated.

It has been discovered that the NSAID prodrug compositions can be an organogel composition and are particularly useful for topical administration of NSAID prodrugs. One such type of organogel useful herein is a lecithin organogel obtained by adding small amounts of water to a solution of lecithin in organic solvents. Generally, lecithin organogels are prepared at room temperature by, for example, dissolving lecithin in an organic solvent and adding enough water while stirring to obtain the a gel with a desired viscosity. One or more NSAID prodrugs can be dissolved in the organic solvent prior to the addition of lecithin.

Useful organic solvents include a hydrocarbons, ethers, amines, and esters. Optionally, the organic solvent is a fatty acid ester such as isopropyl palmitate or isopropyl myristate.

Optionally, the organogel is a pluronic organogel. Optionally the pluronic surfactant is a block copolymers of ethylene oxide and propylene oxide. The pluronic can be added to the water (which can optionally have a drug dissolved in it) solution prior to its addition to the organic solvent/lecithin solution. By way of example, pluronics are typically incorporated in organogels to stabilize the gel matrix wherein the lecithin ingredient is not of high purity.

Furthermore, in organogels the organic solvent can be reduced or replaced by an NSAID prodrug ester. This allows compositions to be prepared at a higher total NSAID concentration. Such compositions are also useful to solubilize an additional drug of poor water solubility.

NSAID prodrugs can be formulated in to an alcohol-free composition of the phospholipids/ polyoxyethylenepolyoxypropylene copolymer type. Moreover, the phospholipid concentration can be reduced or replaced by the NSAID prodrug. This provides for a composition with a useful concentration of NSAID prodrug, a useful viscosity, yet does not deposit substantial amounts of inert ingredient on the skin. Moreover, for some local inflammatory disorders, phospholipids deposited on the skin can have a soothing or even therapeutic effect (e.g., burn from UV exposure).

Oil-in-water (o/w) emulsions are useful compositions for NSAID prodrugs. Such compositions are made of an oil phase, a water phase, and an emulsifier. The oil phase is a useful solvent for the NSAID prodrug as well as other hydrophobic drugs and/or excipients. The water phase can usefully solubilize hydrophilic drugs and/or excipients. Optionally, the solvent for the NSAID prodrug is reduced or replaced by the NSAID prodrug if it is a liquid NSAID prodrug. By way of example, typical emulsifiers are nonionic or anionic surfactants as polyoxyethylene 20 sorbitan trioleate (polysorbate 85), sorbitan monolaurate, polyoxyethylene 4 lauryl ether sodium stearate, and the like. Oil-in-water emulsions are especially beneficial for NSAID prodrugs because one skilled in the art is able to adjust the oil/water ratio to provide sufficient drug solubilization and, at the same time, optimal drug delivery (i.e., movement of the drug from the formulation into the skin).

Methods of Treatment

Herein described is a method of treating a local inflammatory disorder comprising topically administering to a subject in need thereof an NSAID alkyl ester wherein the NSAID is other than naproxen, and wherein the subject is a mammal other than a rodent.

There is also described a method of treating a local inflammatory disorder comprising topically administering to a subject in need thereof an NSAID alkyl ester wherein the NSAID is other than naproxen, and wherein the subject is a human, a livestock animal, or a companion animal

A method of treating an inflammatory epidermal disorder described herein comprises topically administering to a subject in need thereof, an NSAID prodrug , wherein the NSAID prodrug is a phenylacetic acid-type NSAID alkyl ester.

There is also described a method of treating an inflammatory skin disorder comprising topically administering to a subject in need thereof, an NSAID prodrug, wherein the NSAID prodrug is a phenylacetic acid-type NSAID alkyl ester and wherein the subject is a human, a livestock animal, or a companion animal

There is also described a method of treating a local inflammatory disorder comprising topically administering to a subject in need thereof an NSAID prodrug, wherein the NSAID prodrug is an NSAID 1-3 carbon alkyl ester and wherein the subject is a human, a livestock animal, or a companion animal.

Optionally, the local inflammatory disorder is a skin disorder or optionally, an epidermal skin disorder. Optionally, the local inflammatory disorder is psoriasis, folliculitis, PFB, and/or dermatitis.

Alcoholic Gels

Also described herein, are alcoholic gel compositions useful for administering a topically active drug. Optionally, the topically active drug is an NSAID.

Such a composition may comprise:

(1) one or more alcoholic solvents in an amount of about 10% to about 90%,

(2) one or more topically active drugs in a total amount of about 0.001 % to about 25%, and
(3) a polymeric thickener in an amount of about 0.05% to about 5%, wherein the topically active drug is substantially dissolved in the one or more alcoholic solvents.

One or more keratolytic agents including α- and β-hydroxycarboxylic acids and β-ketocarboxylic acids are present in the present compositions at a total keratolytic agent concentration amount of about 0.015% to about 25%, or about 0.05% to about 10%, or about 0.05% to about 5%, or about 0.05% to about 2%. Keratolytic agents useful in alcoholic gel compositions of the present invention are described further herein below.

As shall be readily seen in examples herein, it has been surprisingly discovered that such a keratolytic agent can be used in the present invention at a concentration effective to stabilize the composition with regards to pH and viscosity. One such stabilizing keratolytic agent is salicylic acid, and an exemplary viscosity- and/or pH-stabilizing amount is about 0.05% to about 25%, or about 0.05% to about 10%, or about 0.05% to about 5%, or about 0.05% to about 2%.

Optionally, the keratolytic agent is present in a pH-stabilizing amount.

Optionally, the keratolytic agent is present in a viscosity-stabilizing amount.

Optionally in addition to α-and β-hydrocarboxylic and β-ketocarboxylic acids a further keratolytic agent is selected from the group consisting of salts, amides or esters thereof.

Optionally, the keratolytic agent is a salicylate.

In one embodiment, the polymeric thickener is a polyacrylic thickener. It is surprisingly now discovered that alcoholic gels of the present invention that comprise a polyacrylic thickener provide a therapeutically beneficial pH and viscosity, with a reduced requirement for added alkalinizing agent or without requiring any neutralization step in the process of making the composition. This is contrary to conventional teachings in the art of polyacrylic acid polymeric thickeners. For example, see Noveon technical bulletin which states "target a pH range between 7.3-7.7" and "The key to formulating a hydroalcoholic gel with CARBOPOL® polymers is choosing the correct neutralizing agent. Because the solubility of the CARBOPOL® salt changes with increased alcohol levels, it is necessary to use specific neutralizing agents for specific hydroalcoholic blends." (See Noveon TDS 255 Revised 12/99.)

As will become apparent in examples herein, compositions are now provided herein with therapeutically beneficial pH and viscosity values, yet having reduced or no alkalinizing agent by selection of alcoholic solvent and concentration, active drug and concentration, polyacrylic thickener and concentration, and water concentration. Without being bound by theory, the inventors have evidence to support their theory that novel interactions between a carboxylic acid of the active drug, charge of a polymeric thickener (e.g., acetate), and alcoholic solvent and water concentrations result in attaining rheological properties suitable for topical administration..

The compositions of the present invention are generally acidic and have a pH of from about 3.0 to about 6.5, optionally from about 4.0 to about 5.5, or optionally from about 4.3 to about 5.0.

A composistion described herein comprises:

(1) one or more alcoholic solvents in an amount of about 10% to about 90%,
(2) one or more topically active drugs in a total amount of about 0.001 % to about 25%, and
(3) a polymeric thickener in an amount of about 0.05% to about 5%, wherein the topically active drug is substantially dissolved in the one or more alcoholic solvents, wherein the topically active drug is substantially dissolved in the one or more alcoholic solvents, wherein the composition has a viscosity of about 2,000 to about 50,000 cps without the addition of an alkalinizing agent.

In One composition described comprises:

(1) one or more alcoholic solvents in an amount of about 50% to about 70%,
(2) an NSAID in a total amount of about 5% to no more than about 25%, and
(3) a polymeric thickener in an amount of about 0.05% to about 2%, wherein the composition has a viscosity of about 2,000 to about 50,000 cps without the addition of an alkalinizing agent.

In Another composition described herein comprises:

(1) one or more alcoholic solvents in an amount of about 10% to about 90%,
(2) one or more topically active drugs in a total amount of about 0.001% to about 25%, and
(3) a polymeric thickener in an amount of about 0.05% to about 5%, wherein the topically active drug is substantially dissolved in the one or more alcoholic solvents, wherein the composition has a viscosity of about 2,000 to about 50,000, and wherein the compositions contains no alkalinizing agent in an amount more than required to raise the pH of the composition about 2 pH units, or optionally no more than about 1 pH unit, or about 0.5 pH unit.

In one embodiment, a composition comprises an alkalinizing agent at a concentration less than 0.5%. In one embodiment, no alkalinizing agent is added.

In Compositions described herein may be substantially free of alkalinizing agent. The drug is optionally an NSAID and optionally of the phenylacetic acid-type NSAID.

It has been surprisingly discovered that increasing the water concentration in the composition of the present invention (in the presence of an active drug) causes a marked decrease in viscosity. This is in contrast to formulations without active drug where increased water causes an increase in viscosity.

For example, as can be seen in one or more examples herein, a composition comprising an active drug useful in the present invention, about 25% water, 50% isopropanol, and a polymeric thickener has a viscosity unsuitable for effective therapeutic delivery of an active drug. This is in contrast to similar compositions comprising an active drug useful in the present invention, less than about 24% water and more than about 40% ethanol which have a suitable viscosity.

It has also been discovered that superior therapeutic efficacy can result when gel compositions comprise less than about 24% water and about 40% alcoholic solvent or more (e.g., about 40% to about 80%). Such compositions, applied once or twice a day to PFB patients demonstrated efficacy. This is especially remarkable because the study subjects included those that had chronic symptoms unresponsive to other treatments.

In One composition described herein comprises:

(1) one or more alcoholic solvents in an amount of about 10% to about 90%,
(2) one or more NSAIDs in a total NSAID amount of about 0.001 % to no more than about 25%,
(3) a polymeric thickener in an amount of about 0.05% to about 5%, and
(4) water in an amount from 0 to about 30%.

Optionally, the water is in an amount from about 0% to about 20%.

In another composition described herein comprises:

(1) one or more alcoholic solvents in an amount of about 20% to about 95%,
(2) one or more NSAIDs in a total NSAID amount of about 1% to no more than about 25%,
(3) a polymeric thickener in an amount of about 0.05% to about 5%, and
(4) water in an amount from 0% to about 20%.

In One composition described herein comprises: a phenylacetic-type NSAID prodrug ester, a solvent, and a thickening agent wherein promoiety is an amidyl, a thio, or unsubstituted alkyl.

In Another composition described herein comprises an NSAID prodrug, a solvent, and at least one excipient that is a thickener, a humectant, a keratolytic, an oil, an emollient, a surfactant, a preservative, a colorant, a UV blocker, an antioxidant, or a perfume wherein the NSAID prodrug is an unsubstituted alkyl ester of an NSAID other than naproxen.

optionally, the aforementioned compositions contain a humectant.

An alcoholic gel composition may comprise one or more alcoholic solvents in an amount of about 10% to about 90%, an NSAID in a total amount of about .001 % to about 25%, and a polyacrylic thickener in an amount of about 0.05% to about 5%, wherein the one or more keratolytic agents are present in a total keratolytic agent concentration amount of about 0.015% to about 25%, and wherein the NSAID is substantially dissolved in the one or more alcoholic solvents.

In one composition described herein comprises an alcoholic gel composition comprising at least one alcoholic solvent present in a total amount from about 30% to about 90%, at least one NSAID having a carboxylic acid group, and at least one polymeric thickener selected from the group consisting of polyacrylic acid thickeners and alkylhydroxycellulose thickeners present in a total thickener amount of about 0.1% to about 5%, wherein upon storage of the composition, ester formation between the at least one alcoholic solvent and the carboxylic acid group is less than about 0.03% per day. Optionally the pH of the composition is greater than 5.0. Optionally, the composition further comprises a keratolytic agent (e.g., a salicylate) in an amount that inhibits ester formation (i.e., prodrug formation). Optionally the alcoholic solvent is a branched alcohol or an alcohol with four or more carbons.

An alcoholic gel composition described herein comprises at least one alcoholic solvent present in a total amount from about 30% to about 90%, at least one NSAID having a carboxylic acid group, and at least one polymeric thickener selected from the group consisting of polyacrylic acid thickeners and alkylhydroxycellulose thickeners present in a total thickener amount of about 0.1% to about 5%, wherein upon storage of the composition, ester formation between the at least one alcoholic solvent and the carboxylic acid group is greater than about 0.03% per day. Optionally, the composition has a pH of less than about 5. Optionally, the alcoholic solvent is a straight chain with three or fewer carbons.

As shall be readily apparent from the examples herein, when the active drug has a carboxylic acid group and when the alcoholic solvent is a $C_1$-$C_3$ straight alcohol (e.g., methanol, ethanol, or propanol), the alcoholic solvent and the carboxylic acid group react at an accelerated rate to form an ester upon storage of the composition.

When the active drug has a carboxylic acid and when the alcoholic solvent is a branched alcohol or an alcohol with four or more carbons, the rate of ester formation between the alcoholic solvent and the carboxylic acids group upon storage is inhibited compared to a $C_1$-$C_3$ straight chain alcohol

The keratolytic agent as defined above can optionally be used in the present invention at a concentration effective to increase the rate of esterification of the active drug. One keratolytic agent for this purpose is salicylic acid at a concentration of about 0.05% to about 5%, or about 0.05% to about 2.5%, or about 0.1% to about 1.5%, or about 0.1 % to about 1%.

Also, when the active drug has a carboxylic acid group, increasing the pH of the composition decreases the rate of formation of an ester between the alcoholic solvent and the carboxylic acid group upon storage. Lowering the pH increases the esterification rate. An esterification rate-stimulating pH is about 3.5 to about 5.0. An esterification rate inhibiting pH is above about 5 or about 6 or about 7.

Also as shall also be readily apparent from the examples herein, decreasing water concentration results in an increase in prodrug formation upon storage of a gel composition of the present invention when the active drug has a carboxylic acid group and the alcoholic solvent is a $C_1$-$C_3$ straight chain alcohol. An esterification rate-stimulating water concentration is below about 24%, or below about 20%, or below about 17%. An esterification rate-inhibiting concentration of water is at or above about 24%, or above about 30%, or above about 40%.

As shall also be readily apparent from the examples herein, compositions can now be prepared to comprise a topically acceptable alcoholic solvent, a topically active drug having a carboxylic acid group, a prodrug with the chemical structure equivalent to that formed by esterification of the active drug with the alcoholic solvent, and a polymeric thickener, wherein the drug and the prodrug are at concentrations such that upon storage for six months at room temperature, said concentrations are each maintained within about 80% or about 90%.

Alcoholic gel compositions disclosed herein above optionally further comprise one, two, three, or four of the following:

Glycerine (about 0.1% to 15%)
Panthenol (about 0.1% to 15%)
Polysorbate (about 0.1 % to 15%)
Humectant (about 0.1% to about 20%)

Superior Properties

Without being bound by theory, the inventors believe that the present compositions provide an especially effective treatment for local inflammatory disorders because of the coactions of a topically active drug, a polymeric thickener, an alcoholic solvent, a keratolytic agent as defined above and, optionally, water.

The active drug is solubilized in the alcoholic solvent and is able to partially diffuse through the hydrophobic epidermis. Evidence for diffusion is not only demonstrated by diffusion assays disclosed herein, but by a visual absence of drug on the surface of the skin after the gel has penetrated the skin and/or dried (i.e., absence of "ashing"). Moreover, in some embodiments of the present invention, a prodrug is used with increased hydrophobicity (over its active metabolite). The inventors have discovered that such increased hydrophobicity enables increased direct delivery of drug through the follicle opening to a specific therapeutic target (i.e., the epidermal lining of the follicular pore). In some inflammatory skin disorders such as PFB, this is a common site of injury.

The gel properties of the composition allows administration of an increased volume of composition (i.e., more thickly applied), especially when compared to liquid formulations. This provides higher doses of the topically active drug.

Each component of the composition retards evaporation of the alcohol, allowing extended time for the NSAID to be absorbed into the skin after application. This is an improvement over formulations that evaporate quickly leaving greater amounts of the NSAID dried on the surface of the skin.

In One composition described herein contains a relatively high concentration of at least one NSAID ("high NSAID compositions"). For example, a composition can comprise about 1% to about 20%, such as about 2% or about 5%, or about 10%, or about 15%.

A high NSAID composition, when the NSAID is practically insoluble or poorly soluble in water, contains a high concentration of alcohol, for example, about 10% to about 90% or, for example, more than about 20%, or more than about 40%, or more than about 60%.

By way of example, a 15% concentration of an NSAID of the propionic acid derivative type can be formulated in a 60% alcohol composition.

The inventors have discovered that compositions comprising about 5% to about 20% concentration of an NSAID of the propionic acid derivative type and alcohol at a concentration of about 20% to about 60% have an unexpectedly useful pharmacodynamic profile.

The keratolytic agent removes the dead cells from the epidermis including regions around the hair follicles,

sebaceous glands, and sweat glands, further enhancing diffusion of the active drug carried in the alcoholic solvent.

The optional humectant draws water into the epidermis, follicles, and glands and causes them to open up. This coaction facilitates diffusion of the active drug to the therapeutic targets in skin.

The action of a keratolytic agent and/or a humectant in the composition of the present invention is especially beneficial in PFB, where the hair follicle is the site of the skin injury and, therefore, a therapeutic target.

Methods of Treatment

The aforementioned alcoholic gel compositions are useful for treating subjects affected by a local inflammatory disorder by topical application. A local inflammatory disorder can be, for example, a skin disorder. Other examples of disorders that can be usefully treated with compositions of the present invention are set forth below herein.

A subject with PFB is may be treated by topical application of an alcoholic gel comprising an NSAID, an alcoholic solvent, and a polymeric thickener.

Then is described a method of treating a subject comprising topically administering to a subject in need thereof a composition comprising a phenylacetic-type NSAID prodrug ester, a solvent, and a thickening agent wherein promoiety is an amidyl, a thio, or an unsubstituted alkyl and wherein the subject has a condition selected from psoriasis, folliculitis, eczema and dermatitis.

Topically Active Drugs

Described herein are, *inter alia,* one or more topically active drugs. Exemplary topically active drugs include anti-inflammatories (NSAIDs) and salicylates. While some skilled artisans may classify salicylates as NSAIDs, as used herein, the term NSAID does not include salicylates. Accordingly, as used herein, salicylate means a non-NSAID salicylic acid or a derivative of salicylic acid, such as methyl salicylate, sodium salicylate, trifluoroethyl salicylate, diflunisal, etc.

Topically active drugs can also be selected from analgesics, antibacterial agents, antiwrinkle agents, antihistamines, antifungal agents, anesthetics, corticosteroids, glucocorticoids, antivirals (for example, anti-herpetics), and antiallergic compounds. In the description herein, the phrase "the active drug" and the like are use to mean the more awkward phrase "the one or more active drugs."

The one or more active drugs used in the present invention are selected from ibuprofen salt, ibuprofen free acid, and esters thereof.

In one embodiment, the ester is formed by reaction of an active drug of the present invention and the alcoholic solvent.

In one embodiment, the active drug is present in compositions of the present invention at a total active drug amount of about 0.001 % to about 20% of the total composition, optionally 0.5% to about 20%, or from about 5% to about 20%, or from about 10% to about 20%.

Optionally, the active drug is substantially dissolved in the alcoholic solvent, by way of example, about 90% dissolved.

Alcoholic solvent

Alcoholic gel compositions of the present invention comprise, *inter alia*, one or more alcoholic solvents.

Alcoholic solvents of the present invention are selected from topically acceptable, monohydric or polyhydric alcohols. Alcoholic solvents of the present invention may be present in a total alcohol amount of about 30% to about 80%, optionally from about 40% to about 70%, or optionally from about 50% to about 65%.

Such alcoholic solvents are well known in the art. They may be straight or branched chain and may contain from one to about 14 carbons. They may be unsubstituted or substituted alkyl alcohols. They include, for example, ethanol, isopropyl alcohol, myristoyl alcohol, propylene glycol, glycerin and alkyl glycerol derivatives.

Optionally, the alcoholic solvent is ethanol, isopropyl alcohol, propylene glycol, glycerin, myristoyl alcohol, and mixtures thereof. Optionally, the alcoholic solvent is ethanol. The present invention comprises, *inter alia,* one or more polymeric thickener. In the description herein, the phrase "the polymeric thickener" and the like are use to mean the more awkward phrase "the one or more polymeric thickeners."

Polymeric Thickeners

In one embodiment of the present invention, the polymeric thickener comprises a homo- or copolymer having dissociable side groups on the polymer, such as acetic acid groups.

Optionally, the polymer is a polymer (or copolymer) of polyacrylic acids, such as those sold under the trade name CARBOPOL® (Noveon); polyoxyethylenepolyoxypropylene copolymers (poloxamer), such as available as

LUTROL®, and the like. CARBOPOL®-type resins, such as CARBOPOL® and PEMULEN® (Noveon), are polymers of acrylic acid, crosslinked with polyalkenyl ethers or divinyl glycol. CARBOPOL®-type polymers are flocculated powders of particles averaging about 0.2 micron in diameter. Nonlimiting examples of CARBOPOL® polymers are CARBOPOL® ULTREZ™ 10, CARBOPOL® ULTREZ™ 20, CARBOPOL® ETD™ 2020 and CARBOPOL® ETD™ 2001

Other classes of polymers useful according to the present invention are carboxyvinyl, polyacrylamides, polysaccharides, natural gums (for example, xanthan gum), polyvinlsulfonates, polyalkylsulfones and polyvinylalcohols, or mixtures thereof.

Other classes of polymers useful according to the present invention are alkylhydroxycellulose materials, such as KLUCEL®, commercially available from Hercules (Wilmington, DE).

Nonlimiting examples of alkylhydroxycelluloses useful in the present invention include sodium carboxymethylcellulose, hydroxyethyl cellulose, hydroxypropyl cellulose, hydroxypropylmethylcellulose, and methylcellulose.

Nonlimiting examples of gums useful in the present invention include xanthan gum, sodium carrageenan, sodium alginate, hydroxypropyl guar, gum Arabic (acacia), and gum tragacanth.

The polymeric thickener may be present in the composition of the present invention at a total thickener amount of about 0.1 % to about 5% of the total compositions, optionally 0.5% to about 5%, or from about 1.5% to about 3% of the thickener component.

Keratolytic Agents

The composition of the present invention inculdes one or more keratolytic agents. Keratolytic agents used according to the invention are chosen from α- and β-hydroxycarboxylic or β-ketocarboxylic acids and, in addition there to, may be chosen from salts, amides or esters thereof. More particularly, nonlimiting examples of α-hydroxy acids are glycolic acid, lactic acid, tartaric acid, malic acid, citric acid, mandelic acid and, in general, fruit acids. Nonlimiting examples of β-hydroxy acids are salicylic acid.

Keratolytic agent used in addition to the above-described acids may also be chosen from retinoids (retinoic acid or retinol) and derivatives thereof, benzoyl peroxide, urea, boric acid, allantoin (e.g. glyoxyldiureide or 5-ureidohydantoin) sulfur, resorcinol, and hexachlorophene.

Humectants

Optionally, compositions of the present invention comprise at least one humectant. Humectants useful according to the present invention are hygroscopic compounds that promote retention of water. Nonlimiting examples of such are polyhydric alcohols (e.g., glycerin, propylene glycol, polypropylene glycol, mannitol and sorbitol, and the like) and polyols, such as the polyethylene glycols, fructose, glucose, lactic acid, 1,3 butylene glycol, wheat gluten; macrocytis yyrifera; ceratonia silaqual; hespridin methyl chalocone; dipeptide-2; palmitoyl tetrpeptide-3; palmitoyl pentapeptides, and panthenols.

One or more humectants can optionally be included in the composition in total humectant amount of about 0.1% to about 20%, or about 0.5% to about 10%, or about 1% to about 5%.

Viscosity and pH

Viscosity values that are useful and desirable according to the present invention vary as a function of the indication being treated. For example, where broad coverage (i.e., large areas of skin) or lower levels of drug application are desired, a less viscous composition is advantageous. The less viscous compositions are about 2,000 cps to about 50,000 cps, or about 2,000 cps to about 25,000 cps, or 2,000 cps to about 10,000 cps, or about 5,000 cps to about 15,000 cps. Such less viscous compositions facilitate spreading of applied composition.

Where more restricted coverage or higher levels of drug application are desired, a more viscous composition is advantageous. The more viscous compositions are about 20,000 cps to about 200,000 cps or about 50,000 cps to about 100,000 cps. One skilled in the art will readily be able to increase the viscosity of the present compositions by, for example, increasing the polymeric thickener concentration.

It has also been discovered that such compositions are relatively resistant to viscosity changes upon addition of Alkalinizing agent; for example, less than about 50% viscosity change per pH unit that the composition is alkalinized, or less than about 25%, or less than about 15%.

Optional Components

The compositions of the present invention may also contain optional components which are typically used in topical pharmaceutical and/or cosmetic formulations. These materials, such as solvents, oils, emollients, surfactants,

preservatives, colorants, UV blockers, and perfumes are well known in the art and they are used in the present compositions at their conventional art-established levels for their art-established effects.

Optionally, in other embodiments, it is advantageous to add antioxidants to the compositions of the invention. The antioxidants are advantageously selected from the group consisting of amino acids (e.g., glycine, histidine, tyrosine, tryptophan) and their derivatives; imidazoles, (e.g., urocanic acid) and their derivatives; peptides, such as D,L-carnosine, D-carnosine, L-carnosine and their derivatives (e.g., anserine); carotenoids; carotenes (e.g., alpha-carotene, beta-carotene, lycopene) and their derivatives; chlorogenic acid and derivatives thereof; lipoic acid and its derivatives (e.g., dihydrolipoic acid); aurothioglucose, propylthiouracil and other thiols (e.g., thioredoxin, glutathione, cysteine, cystine, cystamine and their glycosyl, N-acetyl, methyl, ethyl, propyl, amyl, butyl and lauryl, palmitoyl, oleyl, gamma-linoleyl, cholesteryl and glyceryl esters) and their salts; dilauryl thiodipropionate, distearyl thiodipropionate, thiodipropionic acid and its derivatives (esters, ethers, peptides, lipids, nucleotides, nucleosides and salts); and sulfoximine compounds (e.g., buthionine sulfoximines, homocysteine sulfoximine, buthionine sulfones, penta-, hexa-, heptathionine sulfoximine) in very low tolerated doses (e.g., pmol to .mu.mol/kg); and also (metal) chelating agents (e.g., alpha-hydroxy fatty acids, palmitic acid, phytic acid, lactoferrin), alpha-hydroxy acids (e.g., citric acid, lactic acid, malic acid), humic acid, bile acid, bile extracts, bilirubin, biliverdin, EDTA, EGTA and their derivatives; unsaturated fatty acids and their derivatives (e.g., gamma-linolenic acid, linoleic acid, oleic acid); folic acid and its derivatives, ubiquinone and ubiquinol and their derivatives; vitamin C and derivatives (e.g., ascorbyl palmitate, Mg ascorbyl phosphate, ascorbyl acetate); tocopherols and derivatives (e.g., vitamin E acetate); vitamin A and derivatives (vitamin A palmitate); and coniferyl benzoate of benzoin resin; rutinic acid and its derivatives; alpha.-glucosylrutin, ferulic acid, furfurylideneglucitol, carnosine, butylhydroxytoluene, butylhydroxyanisole, nordihydroguaiacic acid, nordihydroguaiaretic acid, trihydroxybutyrophenone, uric acid and its derivatives; mannose and its derivatives; zinc and its derivatives (e.g., ZnO, $ZnSO_4$); selenium and its derivatives (e.g., selenomethionine); stilbenes and their derivatives; (e.g., stilbene oxide, trans-stilbene oxide); and the derivatives (salts, esters, ethers, sugars, nucleotides, nucleosides, peptides and lipids) of said active ingredients which are suitable according to the invention.

The amount of antioxidants (one or more compounds) in the compositions is in an amount of from about 0.001% to about 30%, or from about 0.05% to about 20%, or about 1% to about 10%.

If vitamin E and/or its derivatives are used as the antioxidant or antioxidants, their respective concentrations are advantageously chosen from the range of about 0.001 % to about 10%.

If vitamin A or vitamin A derivatives, or carotenes or their derivatives are used as the antioxidant or antioxidants, their respective concentrations are advantageously chosen from the range of about 0.001% to about 10%.

The compositions may also contain oils, generally at levels of from about 0% to about 5% of the composition. The oils may be present for their emollient effects or can be used as part of an oil/water emulsion composition. The oils which may be used in the present invention are generally partially or poorly soluble in $C_8$ or greater alcohols. Examples of such oils include mineral oils, safflower oil, castor oil, sunflower oil, silicone oil, olive oil, dimethicone, cyclomethicone, triglycerides. Particularly preferred is dimethicone.

Emollients may be included in the compositions of the present invention, generally at levels of from about 0% to about 5%, for the purpose of enhancing both the formulation properties of the compositions (for example, the ability to apply the composition to the skin smoothly), as well as to provide desirable skin feel. Examples of such emollients include silicone materials, such as dimethicones (both cyclic and linear), pantethine derivatives (such as panthenol, pantothenic acid, pantetheine, and pantethine), and allantoin.

The compositions of the present invention may also contain surfactants which generally act to improve the formulation properties of the compositions. Typically, surfactants are included at a concentration of about 0% to about 5% of the composition. Nonionic surfactants are generally the ones used in the present invention, with sorbitol fatty acid esters and alkyl polyethoxylates (for example, $C_8$-$C_{18}$ $(EO)_{4-50}$) being preferred. Examples of surfactants which may be utilized in the present invention include polysorbate 20 and polysorbate 80, both of which have commercial availability.

Optionally, embodiments of the present invention further comprise a UV-absorbing agent such as singular (monomeric) aromatic compounds and/or reflecting pigments such as octyl methoxycinnamate (PARSOL® MCX), benzophenone-3(oxybenzone) and octyl dimethyl PABA.

The composition of the invention may further comprise penetration enhancers for improved transepidermal or percutaneous delivery of drug. The penetration enhancers suitable for the present invention include terpenes, terpene alcohols, essential oils, surfactants, and the like. Some such examples include d-limonene, terpinen-4-ol, menthone, 1,8-cineole, 1-pinene, alpha-terpineol, carveol, carvone, pulegone, eucalyptol, peppermint oil, sorbitan esters, polysorbates, sodium lauryl sulfate, and the like.

Compositions With Other Solvents

The Also described herein are alcohol-free or reduced alcohol compositions useful for treating inflammatory skin disease. The composition may comprise a poorly water-soluble or practically water-insoluble NSAID formulated in

the absence of alcohol. One such composition is an organogel, for example, a lecithin organogel obtained by adding small amounts of water to a solution of lecithin in organic solvents. One or more NSAIDs can be dissolved in the organic solvent.

Organic solvents useful herein include, as nonlimiting examples, hydrocarbons, ethers, amines, and esters. Optionally, the organic solvent is a fatty acid ester such as isopropyl palmitate or isopropyl myristate. Optionally, the organogel of the present invention is a pluronic organogel. It has further been discovered that NSAIDs of the present invention can be formulated in to an alcohol-free composition in a phospholipids/ polyoxyethylenepolyoxypropylene copolymer composition. This provides for a composition with a useful concentration of an NSAID, a useful viscosity, yet does not deposit substantial amounts of inert ingredient on the skin. Moreover, in for some local inflammatory disorders, phospholipids deposited on the skin can have a soothing or even therapeutic effect (e.g., burn from UV exposure).

Oil-in-water (o/w) emulsions are useful compositions for NSAIDs. The oil phase is a useful solvent for the NSAID as well as other hydrophobic drugs and/or excipients. The water phase can usefully solubilize hydrophilic drugs and/or excipients. Oil-in-water emulsions are especially beneficial for NSAIDs because one skilled in the art is able to adjust the oil/water ratio to provide sufficient drug solubilization and, at the same time, optimal drug delivery (i.e., movement of the drug from the formulation into the skin).

PFB formulations

There is described a method of treating PFB comprising applying to the skin of a subject in need thereof, a composition comprising one or more alcoholic solvents in an amount of about 10% to about 90%, one or more NSAIDs in a total amount of about 0.001 % to about 25%, and a polymeric thickener in an amount of about 0.05% to about 5%.

Another method described of treating PFB comprises applying to the skin of a subject in need thereof a composition comprising one or more alcoholic solvents in an amount of about 30% to about 70%, one or more NSAIDs in a total amount of about 1% to less than about 25%, and a polymeric thickener in an amount of about 0.05% to about 5%.

Another method described of treating PFB comprises applying to the skin of a subject in need thereof, a composition comprising one or more alcoholic solvents in an amount of about 30% to about 70%, one or more NSAIDs in a total amount of about 5% to less than about 25%, a polymeric thickener in an amount of about 0.05% to about 5%, and one or more keratolytic agents are present in a total keratolytic agent concentration amount of about 0.015% to about 25%, and wherein the NSAID is substantially dissolved in the one or more alcoholic solvents.

Another method described of treating PFB comprises applying to the skin of a subject in need thereof a composition comprising an NSAID prodrug. Such a composition can be prepared by combining the NSAID prodrug with a dermatologically acceptable excipient.

Local Inflammatory Disorders

The present invention is useful for treating a subject with a local inflammatory disorder such as skin, joints, muscle, and ligaments.

Examples of inflammatory skin disorders that can be effectively treated are disorders of the epidermis and dermis. Nonlimiting examples of such a disorders include eczema and related conditions; insect bites; erythrodenma; mycosis fungoides and related conditions; pyoderma gangrenosum; erythema multiforme; rosacea; onychomycosis; acne, boils, and related conditions; UV damage; psoriasis; folliculitis and related conditions such as in-grown toe and finger nails; acne keloidalis, and boils.

Nonlimiting examples of eczemas useful for treatment atopic eczema, acrodermatitis continua, contact allergic dermatitis, contact irritant dermatitis, dyshydrotic eczema or pompholyx, lichen simplex chronicus, nummular eczema, seborrheic dermatitis, and stasis eczema.

Nonlimiting examples of folliculitis useful for treatment are pseudomonas folliculitis (hot tub folliculitis), barber's itch, tinea barbae, pseudofollieulitis barbae, pityrosporum folliculitis, and herpetic folliculitis.

As used herein, pseudofolliculitis barbae includes pseudofolliculitis of areas other that the beard (barbae). Accordingly, PFB signifies a condition of the skin (or area of the skin) wherein inflammation results from physical trauma caused, at least in part, from hair growth. Accordingly, PFB can affect men with curly hair who shave their faces; women with hirsutism who shave or wax their faces; subjects with curly or sharp-tipped hair who shave their legs, arm pits, and the so-called bikini areas (i.e., pubic region, upper thighs, etc.); as well as individual who develop hair-induced skin inflammation even in the absence of shaving (e.g., ingrown hairs).

PFB subjects can also be treated with the composition of the present invention in combination with other treatments or activities such as shaving, laser treatment, waxing (for hair removal), or depilatory treatment.

The present composition is useful for treating a subject with local pain, for example pain resulting from stimulation of nociceptors in the skin, bones, joints, and muscles. One skilled in the art will readily recognize that many or most of the aforementioned local inflammatory disorders further comprise a pain component resulting from stimulation of nociceptors in the skin. Nonlimiting examples of such pain that result from stimulation ofnociceptors in bones, joints, and muscles usefully treated by the composition of the present invention are arthritis, muscle damage, surgery of bones, joints, and muscles, fibromyalgia, neuropathy, and muscle cramps. The composition of the present invention can also reduce the inflammatory response associated with arthritis.

Delivery Systems and Storage Vessels

Also described is a delivery system (including a storage device) useful for delivering any of the compositions of the present invention.

Delivery system useful for compositions of the present invention include a pump dispenser, jar, spray bottle, wipes, shaving razors adapted for gel delivery, pouch, tube, roll-on, squeeze bottles, aerosol containers, flexible articles intended to be worn on the skin (impregnating said composition into a fibrous or nonfibrous matrix, dermal patch, adhesive tape, etc.).

Suitable propellants for compositions in an aerosol container are the customary known readily-volatile liquefied propellants, for example, hydrocarbons (propane, butane, isobutane) or compressed air.

Examples

The dermatologically acceptable composition of the present invention is made in a conventional manner as exemplified herein.

The composition of the present invention is used for the topical delivery of topically active drug to the skin of a human or animal patient in need of such treatment. Specifically, a safe and effective amount of the composition is applied to the skin at the site where treatment is required. The composition can be used to provide an analgesic or anti-inflammatory effect to the patient by applying a safe and effective amount (e.g., from about 0.002 to about 0.01g/cm$^2$) of a composition wherein the pharmaceutical active is an nonsteroidal anti-inflammatory material, such as ibuprofen.

The following examples are intended to exemplify the compositions of the present invention, as well as their manufacture and their use. The examples are not intended to be limiting of the scope of the present invention.

Example 1 (for reference)

A composition having the following components and properties is made using conventional techniques:

| Component | Amount |
|---|---|
| CARBOMER® ULTREZ™ 10 | 2.5% |
| Ethanol | 55-65% |
| Ibuprofen | 5-18% |

The pH of the final gel is from 3.5 to 4.8. The viscosity of the gel is from 1,200 cps to 75,000 cps.

The composition is made in the following manner:

a) dissolve all alcohol soluble ingredients in the ethanol;
b) add optional liquid components;
c) in a separate vessel, optionally add water and water soluble components and stir until dissolved;
d) combine the optional water/water soluble components to the alcohol solution;
e) add the CARBOMER® slowly with agitation and allow CARBOMER® to hydrate for 18 hours.

When this composition is applied to PFB lesions, in an amount of about 0.005g/cm$^2$, effective treatment of the PFB is seen over a period of several days.

Example 2 (for reference)

Another formulational example is a composition comprising:

a) about 1 to about 40% isopropyl alcohol
b) about 20 to about 50% ethanol

c) 0.01 to about 0.05% safflower oil
d) 5 to about 10% of anesthetic agent
e) 1 to 1.5% thickening agent such as KLUCEL®
f) water qs to 100%

Example 3 (for reference)

Another formulational example is a composition comprising:

a) about 49 to 73% ethanol
b) about 1 to 4% glycerin
c) about 1 to 3% polysorbate 80
d) about 1 to 10% acetaminophen
e) about 0.01 to 0.1% oleyl alcohol
f) 2 to 4% CARBOPOL® 981
g) water qs to 100%

Example 4

Compositions were prepared as shown in Table 1, with and without the active drug ibuprofen ("IBU"). Four different polymeric thickeners were used, namely ULTREZ™ 10, ULTREZ™ 20, 980 (Noveon), and 981 (Noveon). As is shown in Table 2, compositions with 15% ibuprofen show a substantially lower viscosity than the similar composition without an active drug. This was the similar finding for compositions made with each of the polyacrylic polymeric thickeners. We conclude that hydroalcoholic gels of the present invention, when containing a substantial amount of an active ingredient (e.g., 5-20%) and a polyacrylic thickener, have superior viscosity for dermatalogic application without the need of added alkalinizing agent (neutralization).

Table 1a. Compositions

| | Composition 1 a (+ IBU) | Composition 1b (- IBU) |
|---|---|---|
| Component | % W/W | % W/W |
| Ibuprofen | 15 | 0 |
| Ethanol | 57.33 | 57.33 |
| Glycerin | 3 | 3 |
| D-Panthenol | 0.15 | 0.15 |
| Polysorbate 20 | 2 | 2 |
| Propylene Glycol | 2 | 2 |
| Salicylic Acid | 0.15 | 0.15 |
| Polymeric thickener | 2.5 | 2.5 |
| Water | 17.87 | 32.87 |

Table 2. Viscosity

| | Viscosity (cps) | |
|---|---|---|
| Thickening Agent | No IBU | 15% IBU |
| ULTREZ™ 10 | 37,500 | 11300 |
| ULTREZ™ 20 | 42,300 | 20000 |
| 980 ™ (Noveon) | 31,900 | 10700 |

(continued)

| | Viscosity (cps) | |
|---|---|---|
| Thickening Agent | No IBU | 15% IBU |
| 981 ™ (Noveon | 23,800 | 11590 |

Example 5

Compositions were prepared as shown in Table 3. As is shown in Table 4, the compositions comprising 15% ibuprofen and 2.5% polymeric thickener show a substantially lower viscosity than the similar composition without an active drug and comparable to the composition with no active drug and 1.5% polymeric thickener.

Table 3. Compositions

| | Composition 3a | Composition 3b | Composition 3c |
|---|---|---|---|
| Component | % W/W | % W/W | % W/W |
| Ibuprofen | 0 | 0 | 15 |
| Ethanol | 60.35 | 71.85 | 57.33 |
| Glycerin | 3 | 3.57 | 3 |
| D-Panthenol | 0.15 | 0.18 | 0.15 |
| Polysorbate 20 | 2 | 2.38 | 2 |
| Propylene Glycol | 2 | 2.38 | 2 |
| Salicylic Acid | 0.15 | 0.18 | 0.15 |
| ULTREZ™ 10 | 2.5 | 1.78 | 2.5 |
| Water | 29.85 | 17.68 | 17.87 |

Table 4: Viscosity

| Composition | Ibuprofen % | ULTREZ™10 % | Avg Viscosity (cps) |
|---|---|---|---|
| 3a | 0 | 2.5 | 22900 |
| 3b | 0 | 1.78 | 7600 |
| 3c | 15 | 2.5 | 7600 |

Example 6

Compositions were prepared according to Table 5 and viscosity was measured. As shown in Table 6, decreasing the amounts of water resulted in an increase in viscosity. Unexpectedly, a further decrease in water from 25% to 18%, when combined with the addition of 15% ibuprofen (free acid) resulted in a desirable viscosity of 11,300 cps. Thus, a dermatologic composition can be prepared according to the present invention with low water content (e.g., about 5% to about 20%) and no additional alkalinizing agent.

Table 5. Compositions

| | Composition 5a | Composition 5b | Composition 5c | Composition 5d |
|---|---|---|---|---|
| Ingredient | % W/W | % W/W | % W/W | % W/W |
| EtOH | 57.33 | 63.71 | 59.11 | 57.33 |

(continued)

| | Composition 5a | Composition 5b | Composition 5c | Composition 5d |
|---|---|---|---|---|
| Ingredient | % W/W | % W/W | % W/W | % W/W |
| Active | 15.00 | 0.00 | 0.00 | 0.00 |
| Glycerin | 3.00 | 3.32 | 3.10 | 3.00 |
| D-panthenol | 0.15 | 0.17 | 0.15 | 0.15 |
| Salicylic Acid | 0.15 | 0.17 | 0.15 | 0.15 |
| Polysorbate 20 | 2.00 | 2.22 | 2.06 | 2.00 |
| Propylene Glycol | 2.00 | 2.22 | 2.06 | 2.00 |
| Total Water | 17.87 | 25.41 | 30.79 | 32.87 |
| ULTREZ™ 10 | 2.50 | 2.78 | 2.58 | 2.50 |
| Water/EtOH | 31.2 | 40.0 | 52.1 | 57.3 |

Table 6. Viscosity

| Composition | % Active drug (Ibuprofen) | % Water | pH | Avg Viscosity (cps) |
|---|---|---|---|---|
| 5a | 15 | 18 | 3.68 | 11300 |
| 5b | 0 | 25 | 3.83 | 43800 |
| 5c | 0 | 31 | 3.60 | 37900 |
| 5d | 0 | 33 | 3.39 | 37500 |

Example 7

The effect of differing concentrations of water and pH in the present compositions on viscosity was examined. Compositions were prepared according to Table 7.

Table 7. Compositions

| Ingredient | 7a | 7b | 7c | 7d |
|---|---|---|---|---|
| EtOH | 57.33 | 57.33 | 50.75 | 50.35 |
| Active | 15.00 | 15.00 | 15.00 | 15.00 |
| Glycerin | 3.00 | 3.00 | 3.00 | 3.00 |
| D-panthenol | 0.15 | 0.15 | 0.15 | 0.15 |
| Salicylic Acid | 0.15 | 0.15 | 0.15 | 0.15 |
| Tween 20 | 2.00 | 2.00 | 2.00 | 2.00 |
| Propylene Glycol | 2.00 | 2.00 | 2.00 | 2.00 |
| Water | 17.87 | 17.87 | 24.45 | 24.85 |
| ULTREZ™ 10 | 2.50 | 2.50 | 2.50 | 2.50 |
| Water/alcohol | 31.2 | 31.2 | 48.2 | 49.4 |
| pH | 3.68 | 5 | 5 | 5 |
| Alkalinizing agent added | none | diisopropylamine | diethylamine | diisopropylamine |
| Viscosity (cps) | 11600 | 12700 | 7500 | 4000 |

As can be readily observed in Table 7, in a composition substantially similar to Composition 1a but with 18% water and 57% ethanol, adjusting the pH through addition of diisopropylamine results in a modest increase in viscosity. However, in a composition substantially similar to Composition 1a but with 24% water and ~50% ethanol, adjusting the pH to 5.5 through addition of diisopropylamine unexpectedly results in a substantial decrease in viscosity when compared to a similar composition adjust to pH 5.0 with diethylamine. Hence, in compositions of the present invention, decreasing the water to ethanol ratio (e.g., less than 50%) unexpectedly stabilizes viscosity (i.e., results in less pH effects on viscosity).

Example 8

The effect of two different alcoholic solvents on viscosity was tested in the presence and absence of the active ibuprofen. Compositions were prepared according to Table 8. As shown in Table 9, viscosity in the compositions is greater with the ethanol solvent than with the isopropanol solvent. Moreover, addition of 15% active results in a marked decrease in viscosity.

Table 8. Compositions

| | Composition 8a | Composition 8b | Composition 8c | Composition 8d |
|---|---|---|---|---|
| Ingredient | 57% EtOH, ULTREZ™10 | 60% IPA, ULTREZ™10 | 57% EtOH, ULTREZ™20 | 60% IPA, ULTREZ™20 |
| EtOH | 57.33 | 0.00 | 57.33 | 0.00 |
| Isopropyl Alcohol | 0.00 | 60.00 | 0.00 | 60.35 |
| Active | 0.00 | 0.00 | 15.00 | 15.00 |
| Glycerin | 3.00 | 3.00 | 3.00 | 3.00 |
| D-panthenol | 0.15 | 0.15 | 0.15 | 0.15 |
| Salicylic Acid | 0.15 | 0.15 | 0.15 | 0.15 |
| TWEEN® 20 | 2.00 | 2.00 | 2.00 | 2.00 |
| Propylene Glycol | 2.00 | 2.00 | 2.00 | 2.00 |
| Water | 17.87 | 15.20 | 17.87 | 14.85 |
| ULTREZ™ | 2.50 | 2.50 | 2,50 | 2.50 |

Table 9. Viscosity

| Composition | % Active drug (Ibuprofen) | Avg Viscosity (cps) |
|---|---|---|
| 8a | 0 | 37500 |
| 8b | 0 | 27000 |
| 8c | 15 | 11600 |
| 8d | 15 | 3200 |

Example 9

The effect of varying solvent and polymeric thickeners on viscosity was tested in compositions prepared according to Table 10.

Table 10. Compositions

| Ingredient | 10a | 10b | 10c | 10d |
|---|---|---|---|---|
| EtOH | 57.33 | 0.00 | 57.33 | 0.00 |

(continued)

| Ingredient | 10a | 10b | 10c | 10d |
|---|---|---|---|---|
| Isopropyl Alcohol | 0.00 | 60.00 | 0.00 | 60.35 |
| Active | 15.00 | 15.00 | 15.00 | 15.00 |
| Glycerin | 3.00 | 3.00 | 3.00 | 3.00 |
| D-panthenol | 0.15 | 0.15 | 0.15 | 0.15 |
| Salicylic Acid | 0.15 | 0.15 | 0.15 | 0.15 |
| TWEEN™ 20 | 2.00 | 2.00 | 2.00 | 2.00 |
| Propylene Glycol | 2.00 | 2.00 | 2.00 | 2.00 |
| Water | 17.87 | 15.20 | 17.87 | 14.85 |
| ULTREZ™ 10 | 2.50 | 2.50 | 0 | 0 |
| ULTREZ™ 20 | 0 | 0 | 2.50 | 2.50 |

Table 11. Viscosity

| Alcohol Content, CARBOPOL™ | Avg Viscosity (cps) |
|---|---|
| 57% EtOH, Ultrez™10 | 11600 |
| 60% IPA, Ultrez™10 | 3200 |
| 57% EtOH, Ultrez™20 | 20000 |
| 57% IPA, Ultrez™20 | 16200 |

Example 10

The effect of varying solvent and the addition of alkalinizing agent on viscosity was tested in compositions prepared according to Table 12. Hydroalcoholic gel compositions comprising salicylic acid and ethanol attain a higher viscosity than a similar composition comprising salicylic acid and isopropyl alcohol. Moreover, the ethanol/salicylic acid composition showed negligible viscosity change following the addition of alkalinizing agent. When the pH is adjusted one unit for the isopropanol composition, there is a surprising decrease in viscosity.

Table 12. Compositions and Viscosity

| Ingredient | 12a | 12b | 12c | 12d |
|---|---|---|---|---|
| EtOH | 57.33 | 0.00 | 57.33 | 0.00 |
| Isopropyl Alcohol | 0.00 | 60.00 | 0.00 | 60.35 |
| Active | 15.00 | 15.00 | 15.00 | 15.00 |
| Glycerin | 3.00 | 3.00 | 3.00 | 3.00 |
| D-panthenol | 0.15 | 0.15 | 0.15 | 0.15 |
| Salicylic Acid | 0.15 | 0.15 | 0.15 | 0.15 |
| TWEEN™ 20 | 2.00 | 2.00 | 2.00 | 2.00 |
| Propylene Glycol | 2.00 | 2.00 | 2.00 | 2.00 |
| Water | 17.87 | 15.20 | 17.87 | 14.85 |
| ULTREZ™ 10 | 2.50 | 2.50 | 2.50 | 2.50 |
| | | | | |

(continued)

| Ingredient | 12a | 12b | 12c | 12d |
|---|---|---|---|---|
| pH | 3.68 | 4.07 | 5.0 | 5.0 |
| viscosity | 11600 | 3200 | 11700 | 2700 |

Example 11

The effect of varying solvent concentrations and pH on viscosity was tested in compositions prepared according to Table 13. We conclude that hydroalcoholic gels of the present invention, when containing a substantial amount of an active ingredient (e.g., 5-20%), an amount of isopropanol sufficient to dissolve the dermatologic active ingredient, and a polyacrylic thickener, have a useful viscosity for dermatologic application without the need of added alkalinizing agent (neutralization). Such compositions, when water content is greater than about 50%, can be pH adjusted to 5.0 and maintain superior viscosity for dermatologic compositions.

Table 13. Compositions

| Ingredient | 13a | 13b | 13c |
|---|---|---|---|
| pH | pH 4.07 | pH 5.0 | pH 5.0 |
| Isopropyl Alcohol | 60.00 | 60.00 | 50.35 |
| Ibuprofen | 15.00 | 15.00 | 15.00 |
| Glycerin | 3.00 | 3.00 | 3.00 |
| D-panthenol | 0.15 | 0.15 | 0.15 |
| Salicylic Acid | 0.15 | 0.15 | 0.15 |
| Tween 20 | 2.00 | 2.00 | 2.00 |
| Propylene Glycol | 2.00 | 2.00 | 2.00 |
| Water | 15.20 | 15.20 | 24.85 |
| Ultrez™ 10 | 2.50 | 2.50 | 2.50 |
| Viscosity (cps) | 3159 | 2699 | 120 |

Example 12

Composition 1a was prepared with or without 0.15% salicylic acid (SA) (with the difference made up with water addition) and tested for stability of pH and viscosity with storage time. The salicylic acid-containing composition showed better stability of viscosity within 15% of initial values (Figure 1) and pH (Figure 2).

The initial phase (up to 4 weeks) shows about 10% greater variations of pH when no salicylic acid present. From day 28 through 78, while the means for compositions with and without salicylic acid were similar (3.96 vs. 3.90, respectively), the standard deviations for the salicylic acid-containing composition was half that of compositions in the absence of salicylic acid (0.08 vs. 0.16, respectively).

Figure 3 shows a plot of pH vs. viscosity for each of the samples from Figures 1 and 2. This figure clearly shows that in compositions with salicylic acid, viscosity is more stable as a function of pH. Hence, 0.15% is a viscosity and pH-stabilizing concentration of salicylic acid.

Example 13

The effect of various active drugs on viscosity of compositions of the present invention was examined. Compositions were prepared according to Table 14 and viscosity quantified.

Table 14. Viscosity

| Active Drug | Avg Viscosity cps | Delta Viscosity | Delta Viscosity Normalized to % Active Drug | |
|---|---|---|---|---|
| Placebo | 42300 | 0 | 0 | |
| 10% Ibuprofen | 32500 | -9844 | -984 | |
| 10% Acetaminophen | 53000 | 10656 | 1066 | for reference |
| 10% Ketoprofen | 52000 | 9656 | 966 | for reference |
| 10% Aspirin | 40000 | -2344 | -234 | for reference |
| 10% Flufenamic Acid | 31400 | -10944 | -1094 | for reference |
| 2.5% Sulindac | 40600 | -1744 | -174 | for reference |
| 2.5% Phenylbutazone | 40300 | -2044 | -818 | for reference |
| 2.5%Furosemide | 38100 | -4244 | -1698 | for reference |
| 3% Naproxen | 38500 | -3844 | -1538 | for reference |
| 2.5% Phenacetin | 41000 | -1344 | -448 | for reference |

Table 14 also shows that addition of an active drug NSAID to a composition of the present invention can cause a positive or a negative effect on viscosity. Addition of ibuprofen had the most marked viscosity-lowering effect.

Figure 4 shows the normalized change in viscosity plotted against the $\log_{10}$ P value. These data indicate that there is a linear relationship between the viscosity change and $\log_{10}$ P with a group of active drugs with a similar acidic group.

Example 14

Absorption and penetration of the topically active drug ibuprofen in topical compositions was studied using excised human skin from elective surgery procedures described in the FDA and AAPS Report of the Workshop on Principles and Practices of *In Vitro* Percutaneous Penetration Studies: Relevance to Bioavailability and Bioequivalence (Pharm. Res. 4:265, 87).

All compositions were spiked with tracer levels ~1.0 μCi/3.2 mg composition dosed per diffusion cell) of [$^3$H]-ibuprofen. A single, clinically-relevant, finite dose (~5 mg composition/cm$^1$) was applied to dermatomed human abdominal skin from elective surgery. Percutaneous absorption was evaluated using this skin mounted on Bronaugh flow-through diffusion cells maintained at a constant temperature of 32 °C by use of recirculating water baths. These cells have an opening with a nominal area of 0.64 cm$^2$ Fresh receptor fluid, PBS containing 0.1 % sodium azide and 1.5% Oleth 20, was continuously pumped under the dermis at a flow rate of 1 ml/hr and collected in 6-hour intervals. Following a 24-hour duration of composition exposure to the skin, composition residing on the skin surface was removed by wiping with two, dry, cotton swabs. To remove any residual composition remaining on the skin surface, the upper layers of the stratum corneum were removed from the epidermis with a single cellophane tape-strip. The remaining epidermis was then physically separated from the dermis and processed for analysis separately. Quantity of radioactivity in the wipes, tape-strip, epidermis, dermis, and receptor fluid samples was determined using liquid scintillation counting techniques.

Gel compositions similar to Composition 1a were prepared with modifications as shown in Table 15; the other gels were purchased, commercial preparations.

Table 15. Compositions

| | 15a | 15b | 15c | 15d |
|---|---|---|---|---|
| IBU | 15 | 10 | 15 | 15 |
| EtOH | 60.2 | 60.2 | 60.2 | 60.2 |
| D-panthenol | 0.15 | 0.15 | - | 0.15 |
| panthenine | | | 0.15 | |
| EDTA | 0.05 | 0.05 | - | 0.05 |

(continued)

| | 15a | 15b | 15c | 15d |
|---|---|---|---|---|
| Salicylic acid | 0.15 | 0.15 | 0.15 | 0.15 |
| ULTREZ™ 10 | 2.5 | 2.5 | 2.5 | - |
| KLUCEL® | | | | 2.5 |

Table 16: Results

| | | Single Tape Strip | Receiver |
|---|---|---|---|
| 10% Boot's Gel | Mean | 3.87 | 3.90 |
| | SD | 2.88 | 2.25 |
| | %CV | 74.22 | 57.62 |
| 5% Ibuleve Gel | Mean | 18.42 | 11.53 |
| | SD | 2.36 | 4.62 |
| | %CV | 12.80 | 40.06 |
| 10% Ibuleve Gel | Mean | 20.71 | 8.15 |
| | SD | 2.62 | 3.80 |
| | %CV | 12.64 | 46.65 |
| 15a | Mean | 13.38 | 5.98 |
| | SD | 11.12 | 2.82 |
| | %CV | 83.09 | 47.22 |
| 15b | Mean | 17.57 | 13.30 |
| | SD | 9.15 | 1.99 |
| | %CV | 52.06 | 14.99 |
| | | | |
| 15c | Mean | 8.77 | 5.89 |
| | SD | 7.22 | 1.69 |
| | %CV | 82.36 | 28.70 |
| 15d | Mean | 67.40 | 6.59 |
| | SD | 6.90 | 3.04 |
| | %CV | 10.23 | 46.15 |

As shown in Table 16 and Figure 5, compositions 16a-d have desirable percutaneous absorption. It should be noted that percutaneous absorption demonstrated in this ex vivo assay is but one factor contributing to delivery of therapeutically effective drug to target areas.

Example 15

It has been discovered that, in one embodiment, compositions of the present invention, upon storage, result in the generation of an ester form of the active ingredient. Such ester formation results from reaction of a carboxylic acid group of the active drug with the alcoholic solvent to form an ester linkage.

HPLC analysis was performed on composition 1a stored for 3 months at 25°C. A new peak i.e. the ester distinct from the ibuprofen peak was detected within the chromatographic profile. The peak showed an elution position considerably later than Ibuprofen and a UV response at 220 nm.

Next, the peak was characterized in terms of retention position, UV spectrum and mass spectroscopy response. In addition, isolates of the peak were collected from the chromatograph system employed for liquid chromatography-mass spectroscopy.

Next, two grams of composition 1a were diluted in twenty-five milliliters of (50:50) water:acetonitrile. The solution was centrifuged and the supernatant collected for analysis.

Chromatography was conducted as follows:

| | |
|---|---|
| Pumps: | Hewlett Packard Model 1100 Binary Systems |
| Solvent A: | Water |
| Solvent B: | Acetonitrile |
| Gradient: | Start 40 % B |
| | Raise to 60% B at 20 minutes |
| | Raise to 90% B at 40 minutes |
| Flow Rate: | 1.0 ml/min |
| Stationary Phase | ZORBAX® CS (4.6 x 150mm) |
| Column Temperature: | 25C |
| Injection volume | 25 L |

Sequential detection was performed by UV absorbance using an HP diode array detector followed by ESI-MS followed by ESI-MS using a Sciex QSTAR®/Pulsar quadrupole-TOF mass spectrometer operating in either the positive and negative ion modes.

Figure 6 illustrates the UV chromatogram (220 nm) following injection of Composition 1a stored 3 months at 25°C using the chromatographic conditions described above. Ibuprofen showed a peak at about 14 minutes and the ester showed a peak at about 32 minutes.

Figure 7a shows the positive ESI mass spectrum for the Ibuprofen peak. The expected (M+H)+ pseudomolecular ion is observed at m/z 207.13 with corresponding $(M+NH4)^+$ and $(M+Na)^+$ pseudomolecular ions at m/z 224.15 and 229.10 respectively. Dimeric cluster ions may be assigned to signals at m/z 430.27 and m/z 435.22. A notable, possible fragment ion also appears at m/z 161.12 consistent with decarboxylation as illustrated below:

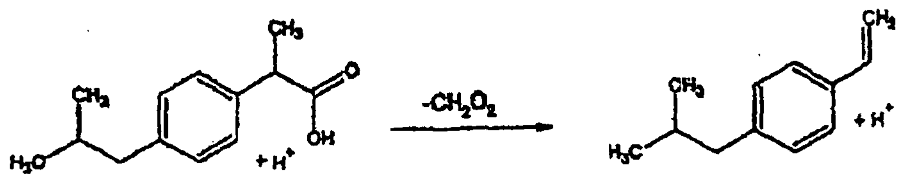


m/z 207 m/z 161

Figure 7b shows the UV spectrum for the Ibuprofen which demonstrates maxima at approximately 220 nm and 265 nm.

Figure 8a shows the positive ESI mass spectrum obtained from the ester. A possible $(M+H)^+$ is observed at m/z 235.15 and, as in the Ibuprofen data, corresponding $(M+NH_4)^+$ and $(M+Na)^+$ pseudomolecular ions may be assigned at *mlz* 254.13 and m/z 257.13 respectively. Of note is the signal at m/z 161.12 consistent with the same fragment ion described for Ibuprofen.

Figure 8b shows the UV spectrum obtained from the ester and is very similar to that obtained for Ibuprofen with maxima at approximately 220 nm and 265 nm.

The data obtained during this study indicate that the ester has (1) a neutral mass of 234.15 Da; (2) a UV spectrum very similar to that of Ibuprofen; (3) retention behavior that suggests it to be considerably more hydrophobic than ibuprofen; (4) no significant negative ion MS response; and (5) a positive ion MS spectrum indicating a shared fragment with ibuprofen.

These data support the identity of the ester being ethylisobutylphenyl-propionate.

Example 16

The effect of alkalinizing agent ("neutralization") on the generation of ester was examined in Composition 1a. As shown in Figure 10, prodrug generation is linear for at least the first 26 days. In the composition without alkalinizing

agent, that rate was approximately 0.05% per day as compared to the lower rate of about 0.025% per day in the neutralized samples.

Example 17

The effect of alkalinizing agent ("neutralization") on the generation of ester was examined in composition 1a in a longer term experiment. Figure 11 shows that generation of ester in the absence of alkalinizing agent is in steady state for at least 100 days.

Example 18

The effect of initial active drug concentration and use of various alkalinizing agents on ester generation (or drug stabilization). Compositions were prepared according to Table 7. As can be seen in Figure 12, alkalinizing agent substantially decrease the rate of ester formation. Moreover, decreasing the concentration of active drug in neutralized compositions substantially decrease the rate of formation. Linear extrapolation of the data indicate that at an initial concentration of 14.8% ibuprofen in neutralized compositions would prevent the formation of the ester.

Example 19

The effect of differing concentrations of water and pH in the present compositions on ester formation rate was examined. As can be readily observed in Table 17, in a composition substantially similar to Composition 1a but with 18% water and 57% ethanol, increasing the pH through addition of diisopropylamine results in a market reduction in the ester formation rate.

When in a composition substantially similar to Composition 1a is made to contain 24% water and ~50% ethanol, there is a further reduction in the ester formation rate. Surprisingly, adjusting the pH to 5.5 through addition of diisopropylamine results in a substantial increase in ester formation rate when compared to a similar composition adjust to pH 5.0 with diethylamine.

Table 17:

| % water | % ethanol | pH | Added alkalinizing agent | % prodrug/day |
|---|---|---|---|---|
| 18 | 57 | 3.68 | none | 0.0516 |
| 18 | 57 | 5.0 | diisopropylamine | 0.0262 |
| 24 | 51 | 5.0 | diethylamine | 0.0078 |
| 24 | 50 | 5.5 | diisopropylamine | 0.0200 |

Example 19

Compositions similar to 1a were prepared and tested for ester formation in the presence or absence of salicylic acid. As shown in Figure 13, salicylic acid increases the rate of ester formation.

Example 21

Compositions containing stabilized concentrations of topically active drug and an ester are prepared according to Table 18.

These compositions are prepared according to equilibrium equation of the esterification process, namely:

$$\text{ACID} + \text{ALCOHOL} = \text{ESTER} + \text{WATER}$$

The equilibrium constant, K, describing the equilibrium state is

$$K = [\text{Ester}][\text{Water}]/[\text{Acid}][\text{Alcohol}]$$

where [] represents "concentration".

These compositions are stored at room temperature for six months and the concentrations of the active drug and the ester are determined. In all cases, the initial concentrations and final concentrations are within 10% of the initial concentrations.

Fresh compositions are also prepared according to Table 18 and stored at 40°C for 30 days and the concentrations of the active drug and the ester are determined. In all cases, the initial concentrations and final concentrations are within 10% of the initial concentrations.

Table 18

| | Alcohol (%) | Water (%) | Temperature | pH | Active drug (%) | Prodrug (%) |
|---|---|---|---|---|---|---|
| 18a | 60 ethanol | 15 | 25 | 4.0 no additional alkalinizing agent | 12 ibuprofen | 3 ibuprofen ethyl ester |
| 18b | 50 ethanol | 24 | 25 | 4.0 no additional alkalinizing agent | 13.5 ibuprofen | 1.5 ibuprofen ethyl ester |
| 18c | 70 ethanol | 5 | 25 | 4.0 no additional Alkalinizing agent | 6 ibuprofen | 9 ibuprofen ethyl ester |
| 18d | 80 ethanol | 5 | 25 | 4.0 no additional alkalinizing agent | 4.5 ibuprofen | 10.5 ibuprofen ethyl ester |
| 18e | 60 ethanol | 15 | 40 | 4.0 no additional alkalinizing agent | 3 ibuprofen | 12 ibuprofen ethyl ester |
| 18f | 50 ethanol | 24 | 40 | 4.0 no additional alkalinizing agent | 9 ibuprofen | 6 ibuprofen ethyl ester |
| 18g | 60 ethanol | 15 | 25 | 5.0 added diisopropanol | 13.2 ibuprofen | 1.8 ibuprofen ethyl ester |

Example 22 (for reference)

PFB efficacy is examined by a 10-week double blind, placebo-controlled, cross-over clinical trial. The investigator performs a quantitative assessment of PFB lesions at the baseline at weekly thereafter. Papules, pustules, and ingrown hairs as defined below are counted and recorded.

The primary objectives of this study are:

- To determine the efficacy of various NSAID compositions applied at various intervals ranging from every other day to twice per day for five weeks in reducing the signs and symptoms of PFB; and
- To determine the safety and tolerability of the various NSAID compositions.

Papules, pustules, and ingrown hairs as defined below are counted and recorded.

Papule: A small solid elevation less than 1.0 cm in diameter.

Pustule: A small, circumscribed elevation of the skin which contains yellowwhite exudates.

Ingrown Hair: A hair that has exited the skin, curved around and reentered the skin, or a hair that has pierced the follicle and is growing under or in the skin.

Lesions are counted on the neck, lower left and right cheeks, and jaw line (beard area). The same qualified physician completes the assessment at each visit. Each assessment is performed independent of previous assessments. Subjects have a total of at least 10 (for moderate) of 2 (for mild) follicular papules, pustules, or ingrown hairs at the Baseline Visit to be admitted to the study.

Inflammatory and/or nodulocystic lesions, erythema, and hyperpigmentation are assessed according to the following six-point Likert (categorical) scale:

0 None: No evidence of active disease.
1 Minimal: Rare noninflammatory lesions present (lesions must be resolving and may be hyperpigmented, though not pink/red). Barely perceptible elevation (discemable by touch only).
2 Mild: Noninflammatory lesions predominate, with few inflammatory papules/pustules. Light red color. Visible but mild elevation. No nodulocystic lesions.
3 Moderate: Some noninflammatory lesions are present with multiple inflammatory lesions evident. Definite lesion redness and elevation. There may or may not be one small nodulocystic lesion.
4 Severe: Highly inflammatory lesions predominate. Deep intense red color. Marked dermal swelling and in duration in widespread areas. There may or may not be a few nodulocystic lesions.
5 Very Severe: Many nodulocystic lesions. Results are recorded in the source document and on the appropriate CRF. The same qualified physician will complete the assessment at each visit. Each assessment should be performed independent of previous assessments. Subjects must have a rating of at least moderate (3) at the Baseline Visit to be admitted to the study.

All subjects are asked to evaluate specific PFB symptoms of itch, pain, and shaving discomfort, as well as the overall condition of their PFB at the Baseline, and weekly thereafter ("Subject’s Assessment of Symptoms").
Subjects complete the following five-point Likert (categorical) scale for each symptom and for overall condition:

0 None: symptom/overall PFB condition absent.
1 Mild: symptom/overall PFB condition present but not particularly bothersome.
2 Moderate: symptom/overall PFB condition present and bothersome, but does not interfere with daily activities.
3 Severe: symptom/overall PFB condition present and bothersome and interferes with some daily activities.
4 Very Severe: symptom/overall PFB condition present and bothersome and prevents many normal daily activities
Each assessment should be performed independent of previous assessments.

Global Assessment of Improvement. Subjects are asked to compare the overall condition of their PFB at the week 2, 4, and 6 visits with the overall condition before treatment using the following five-point Likert (categorical) scale:

2 Overall condition and shaving comfort much better than before treatment.
1 Overall condition and shaving comfort slightly better than before treatment.
0 Overall condition and shaving comfort unchanged, same as before treatment.
-1 Overall condition and shaving comfort slightly worse than before treatment.
2 Overall condition and shaving comfort much worse than before treatment.

Each assessment is performed independent of previous assessments.
After completion, studies are evaluated and indicate that alcoholic gels contain an NSAID of the phenylacetic acid type are effective to reduce severity of PFB in mild, moderate, and severe PFB. Moreover, organogels containing high concentrations of NSAID of the phenylacetic acid type are effective in treatment of PFB with an "every-other-day" application regimen. Test subjects with acne or dermatitis (e.g., contact dermatitis) also report therapeutic efficacy against there indications.
Certain subjects are treated with alcoholic gels containing 5% NSAID prodrug (e.g., ethyl ester of an NSAID of the phenylacetic acid type) and report higher efficacy than subjects treated with an equivalent composition containing the NSAID parent drug (instead of the prodrug).
Certain subjects are treated with organogel containing 10% NSAID of the phenylacetic acid type and report efficacy similar to subjects treated with an alcoholic composition (10% NSAID of the phenylacetic acid type) but report that the organogels have less of a drying effect and cause less stinging of razor cuts.
Certain subjects, in the normal course of their disease, routinely experience more severe inflammation around razor bumps, nodulocystic lesions, erythema, and hyperpigmentation. Such subjects report improvement of such pathologies.

Example 23

Radioactive ($C_{14}$) and nonradioactive ethyl esters and isopropyl esters of ibuprofen and of ketoprofen are synthesized. The esters are made between the hydroxyl group of the carboxylic acid using synthesis of NSAID alkyl ester.
Under N2 atmosphere, a solution of 2-[4-(2-methylpropyl)phenyl]propanoic acid (9.6 gm; 465 m mol) and p-toluene sulfonic acid (1.52 gm, 7.9 mmol) in toluene (100 ml) and ethanol (75 ml) is heated to reflux using a Dean-Stark apparatus for four hours. The solvent is removed under reduce pressure and the residue was taken up in ethanol (100 ml). The solution is extracted with saturated aqueous $NaHC0_3$ solution (2 X 100 ml) and water (2 X 100 ml). The organic layer is dried over anhydrous $Na_2SO_4$, filtered and concentrated, affording 10.4 grams as a clear oil. Similarly, radiolabel ibuprofen ethyl ester is synthesized as above, only the starting material 2-[4-(2-methylpropyl)phenyl]propanoic acid, is $C_{14}$ labeled.

The other NSAID alkyl esters are similarly made. Each is formulated separately at 15% prodrug in 60% alcohol corresponding to the promoiety (i.e., reactant), 1% ULTREZ™ 10, and 24% water. A comparator composition is also prepared with ketoprofen. A placebo is prepared with no active.

The prodrug compositions are tested on PFB subjects according to Example 22 including pharmacokinetic analysis.

Additionally, 0.2 gm of the $C_{14}$-labeled compositions are applied per $cm^2$ of skin of minipigs, and punch biopsies of skin are taken from multiple sites at intervals from 30 seconds to 24 hours after application. Serum samples are also taken at intervals. Results are shown in Table 19.

| Starting material | Reactant | Product | Systemic Levels (1=high, 5=low) | Diffusion Rate (1=high, 5=low) | Efficacy (1=high, 5=low) | |
|---|---|---|---|---|---|---|
| 2-(3-benzoylphenyl) propanoic acid | ethanol | ibuprofen ethylester | 4 | 2 | 2 | |
| 2-(3-benzoylphenyl) propanoic acid | isopropanol | ibuprofen isopropyl ester | 5 | 1 | 1 | |
| 2-[4-(2-methylpropyl) phenyl]propanoic acid | ethanol | ketoprofen ethylester | 3 | 3 | 3 | for reference |
| 2-[4-(2-methylpropyl) phenyl]propanoic acid | isopropanol | ketoprofen isopropyl ester | 3 | 3 | 3 | for reference |
| | | ketoprofen | 2 | 5 | 5 | for reference |
| | | placebo | | | | |

Example 24 (for reference)

Oil in water NSAID prodrug compositions are formulated as illustrated in Table 20.

Table 20

| | A | B | C |
|---|---|---|---|
| Aqueous Phase: | | | |
| Water | 10%-45% | 25%-35% | 20% |
| Alcohol | 10%-30% | 0%-10% | 0%-10% |
| Water-soluble active agent | Yes | Yes | Yes |
| Thickener | <10% | <10% | <10% |

(continued)

| | | | |
|---|---|---|---|
| Oil Phase: | | | |
| Petroleum | 30%-90% | 0%-30% | 0% |
| NSAID prodrug (e.g., ibuprofen ethyl ester) | 10%-90% | 45%-90% | 50% |
| Fatty Acid | 30%-90% | 0%-30% | 0% |
| Surfactant | <15% | <15% | <15% |
| Ibuprofen | - | Yes | Yes |
| Salicylic Acid | Yes | - | Yes |
| | | | |
| | D | E | F |
| Aqueous Phase: | | | |
| Water | 45%-70% | 30%-70% | 53% |
| Alcohol | 0%-10% | 5%-15% | 5% |
| Water-soluble active agent | Yes | Yes | 0% |
| Thickener | <10% | <10% | <5% |
| Oil Phase: | | | |
| Petroleum | 10%-35% | 15%-35% . | 0% |
| NSAID prodrug ester (e.g., ibuprofen ethyl ester) | 10%-40% | 25%-50% | 30% |
| Fatty Acid | 10%-35% | 15%-35% | 0% |
| Surfactant | <15% | <10% | <5% |
| NSAID | Yes | Yes | 5% |
| Salicylic Acid | Yes | Yes | 2% |

Example 25 (for reference)

Compositions are formulated according to Table 20. Each NSAID or NSAID prodrug is formulated four different ways: as an organogel ("A"), as an oil-in-water ("B"), as an alcoholic gel ("C"), and as a phospholipids/polyoxyethylenepolyoxypropylene copolymer composition. The compositions are formulated according to the teaching in the present invention and by consideration of the physicochemical properties of each drug. Each composition is prepared at three pHs: 4.0, 5.0, and 6.0.

Drug concentrations are 15% (if soluble) or at an empirically-determined saturation concentration. Drug absorption, distribution, metabolism and elimination is determined in ex vivo and in vivo animal models.

Efficacy is measured in the contact dermatitis model in the hairless guinea pig (for example, J Dermatol. 1992 Mar;19(3):140-5.), psoriasis in the mouse model overexpressing amphiregulin, Atopic Dermatitis in the Epidermal Interleukin-4 transgenic mouse model, (Journal of Investigative Dermatology Volume 117 Issue 4, Page 977, October 2001), and other models.

All data are analyzed using nonparametric analysis of variance. Models are generated to aid in the selection and optimization of NSAID (and/or NSAID prodrug) and formulation for various inflammatory skin disorders.

| NSAID | Prodrug ester/ether | Formulation |
|---|---|---|
| Bufexamac | methyl | A, B, C, D |
| dicoflenac | ethyl | A, B, C, D |
| etofenamate | isopropyl | A, B, C, D |
| felbinac | n-butyl | A, B, C, D |
| entiazac | palmityl | A, B, C, D |

(continued)

| NSAID | Prodrug ester/ether | Formulation |
|---|---|---|
| fepradinol | 4-(nitrooxy)butyl | A, B, C, D |
| flufenamic | Dimethylformamidyl | A, B, C, D |
| lunoxaprofen | alcoholic xyethyl | A, B, C, D |
| flubiprofen | isopropyloxy | A, B, C, D |
| ibuprofen | lauryl | A, B, C, D |
| indomethacin | isopropyl | A, B, C, D |
| sonixin | isopropyloxy | A, B, C, D |
| Ketoprofen | lauryl | A, B, C, D |
| ketorolac | N-ethyloxy N-propyl N-ethyl amino | A, B, C, D |
| Niflumic | p-alcoholic xyphenylurea | A, B, C, D |
| Oxyphenbutazone | polyethylene glycyl | A, B, C, D |
| piketoprofen | polyethylenyl | A, B, C, D |
| piroxicam | propylene glycoxymercaptoethyl | A, B, C, D |
| suxibuzone | N-ethyloxy, N-propyl, N-ethyl, aminoethyl | A, B, C, D |
| ufenamate | ethyl | A, B, C, D |
| Bufexamac | - - - | A, B, C, D |
| dicoflenac | - - - | A, B, C, D |
| etofenamate | - - - | A, B, C, D |
| felbinac | - - - | A, B, C, D |
| entiazac | - - - | A, B, C, D |
| fepradinol | - - - | A, B, C, D |
| flufenamic | - - - | A, B, C, D |
| lunoxaprofen | - - - | A, B, C, D |
| flubiprofen | - - - | A, B, C, D |
| ibuprofen | - - - | A, B, C, D |
| indomethacin | - - - | A, B, C, D |
| sonixin | - - - | A, B, C, D |
| Ketoprofen | - - - | A, B, C, D |
| ketorolac | - - - | A, B, C, D |
| Niflumic | - - - | A, B, C, D |
| Oxyphenbutazone | - - - | A, B, C, D |
| piketoprofen | - - - | A, B, C, D |
| piroxicam | - - - | A, B, C, D |
| pranoprofen | - - - | A, B, C, D |
| suxibuzone | - - - | A, B, C, D |
| ufenamate | - - - | A, B, C, D |

## Claims

1. A dermatologically acceptable alcoholic gel composition comprising

   (a) at least one alcoholic solvent in a total solvent amount of 10% to 90%,
   (b) one or more NSAID selected from ibuprofen salt, ibuprofen free acid and esters thereof in a total amount of 0.00 1 % to 25%,
   (c) a polymeric thickener in an amount of 0.05% to 5%,
   (d) water in an amount of 0 to 20%, and
   (e) one or more keratolytic agents including, in a total amount of 0.015% to 25%, one or more keratoytic agents selected from $\alpha$- and $\beta$-hydroxylic acid and $\beta$-ketocarboxylic acids, wherein the viscosity of the composition is 2,000 cps to 200,000 cps.

2. The composition of claim 1, wherein the keratolytic agent includes salicylic acid.

3. The composition of claim 1, wherein the NSAID is substantially dissolved in the at least one alcoholic solvent and wherein the composition contains no alkalinizing agent in an amount more than required to raise the pH of the composition about 2 pH units and furthermore wherein the composition has a viscosity of 2,000 to 50,000 cps.

4. The composition of claim 1 or 2 wherein the pH is from 3.0 to 6.5.

5. The composition of claim 1 further comprising from 0% to less than 24% water and the one or more alcoholic solvents in an amount of 40% to 80%.

## Patentansprüche

1. Dermatologisch annehmbare alkoholische Gelzusammensetzung, umfassend

   (a) mindestens ein alkoholisches Lösungsmittel in einer Gesamtlösungsmittelmenge von 10 % bis 90 %,
   (b) ein oder mehrere NSAID, ausgewählt aus Ibuprofensalz, freier Ibuprofensäure und deren Estern in einer Gesamtmenge von 0,001 % bis 25 %,
   (c) ein polymeres Verdickungsmittel in einer Menge von 0,05 % bis 5 %,
   (d) Wasser in einer Menge von 0 bis 20 %, und
   (e) ein oder mehrere keratolytische Mittel, die ein oder mehrere keratolytische Mittel, die aus $\alpha$- und $\beta$-Hydroxysäuren und $\beta$-Ketocarbonsäuren ausgewählt sind, in einer Gesamtmenge von 0,015 % bis 25 % einschließen, wobei die Viskosität der Zusammensetzung 2.000 cps bis 200.000 cps ist.

2. Zusammensetzung nach Anspruch 1, bei der das keratolytische Mittel Salicylsäure einschließt.

3. Zusammensetzung nach Anspruch 1, bei der das NSAID im Wesentlichen in dem mindestens einen alkoholischen Lösungsmittel gelöst ist, und wobei die Zusammensetzung kein alkalisierendes Mittel in einer größeren Menge enthält als es erforderlich ist, um den pH der Zusammensetzung um etwa 2 pH-Einheiten zu erhöhen, und wobei weiter die Zusammensetzung eine Viskosität von 2.000 bis 50.000 cps aufweist.

4. Zusammensetzung nach Anspruch 1 oder 2, bei der der pH 3,0 bis 6,5 beträgt.

5. Zusammensetzung nach Anspruch 1, weiter umfassend 0 % bis weniger als 24 % Wasser und das eine oder die mehreren alkoholischen Lösungsmittel in einer Menge von 40 % bis 80 %.

## Revendications

1. Composition de gel alcoolique acceptable sur le plan dermatologique, comprenant

   (a) au moins un solvant alcoolique en une quantité totale de solvant de 10 % à 90 %,
   (b) un ou plusieurs AINS sélectionnés parmi un sel d'ibuprofène, l'acide libre de l'ibuprofène et ses esters en une quantité totale de 0,001 % à 25 %,

(c) un épaississant polymère en une quantité de 0,05 % à 5 %,
(d) de l'eau en une quantité de 0 % à 20 %, et
(e) un ou plusieurs agents kératolytiques comportant, en une quantité totale de 0,015 % à 25 %, un ou plusieurs agents kératolytiques sélectionnés parmi l'acide α- et β-hydroxylique et les acides β-cétocarboxyliques, dans laquelle la viscosité de la composition est de 2000 cps à 200 000 cps.

**2.** Composition selon la revendication 1, dans laquelle l'agent kératolytique comporte l'acide salicylique.

**3.** Composition selon la revendication 1, dans laquelle l'AINS est sensiblement dissous dans l'au moins un solvant alcoolique et dans laquelle la composition ne contient pas d'agent alcalinisant en une quantité supérieure à ce qui est requis pour augmenter le pH de la composition d'environ 2 unités de pH et, en outre, dans laquelle la composition possède une viscosité de 2000 cps à 50 000 cps.

**4.** Composition selon la revendication 1 ou 2, dans laquelle le pH est de 3,0 à 6,5.

**5.** Composition selon la revendication 1, comprenant en outre de 0 % à moins de 24 % d'eau et le ou les solvants alcooliques en une quantité de 40 % à 80 %.

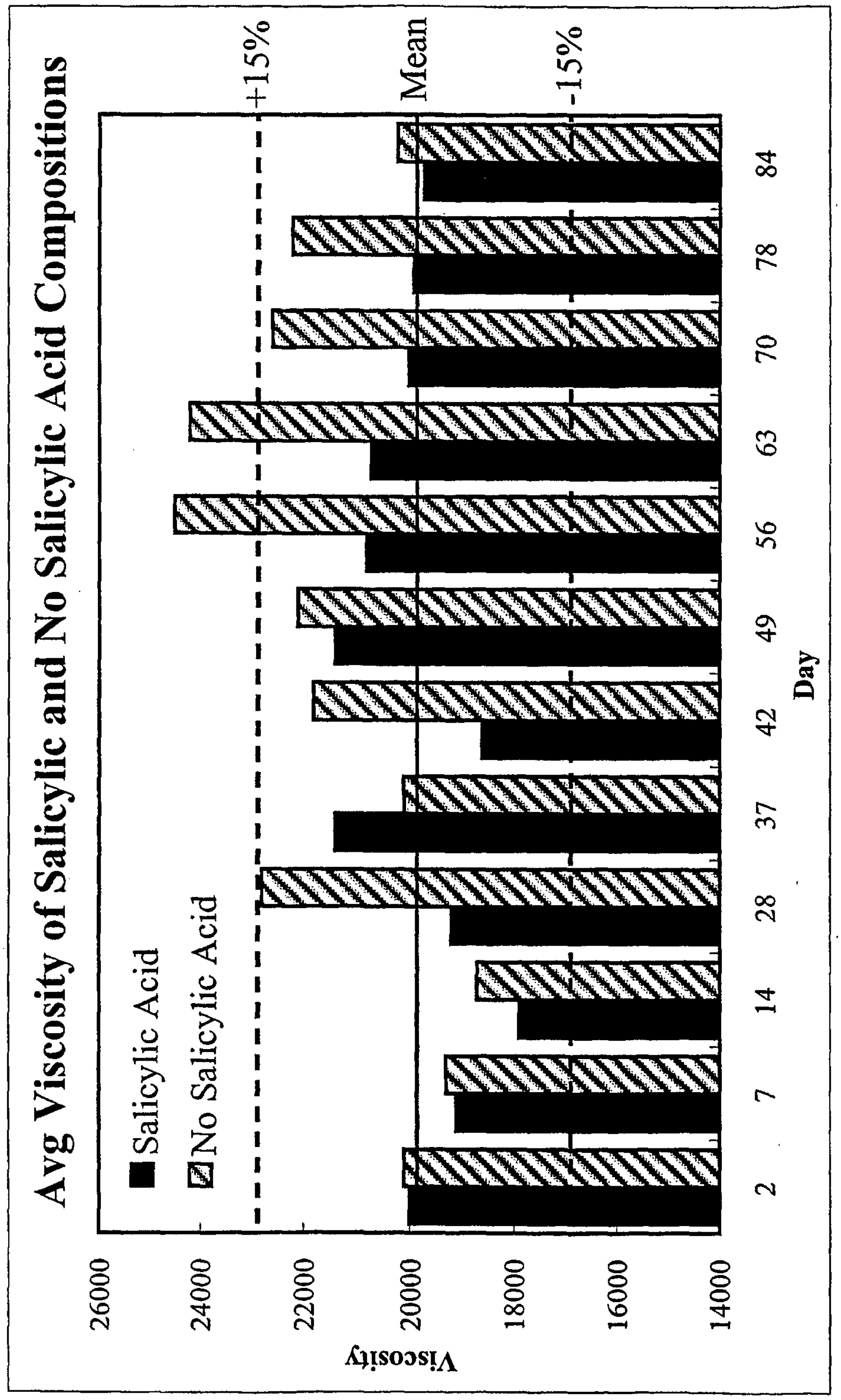
Avg Viscosity of Salicylic and No Salicylic Acid Compositions
Salicylic Acid
No Salicylic Acid
+15%
Mean
-15%
Viscosity
26000
24000
22000
20000
18000
16000
14000
Day
2
7
14
28
37
42
49
56
63
70
78
84

Figure 1

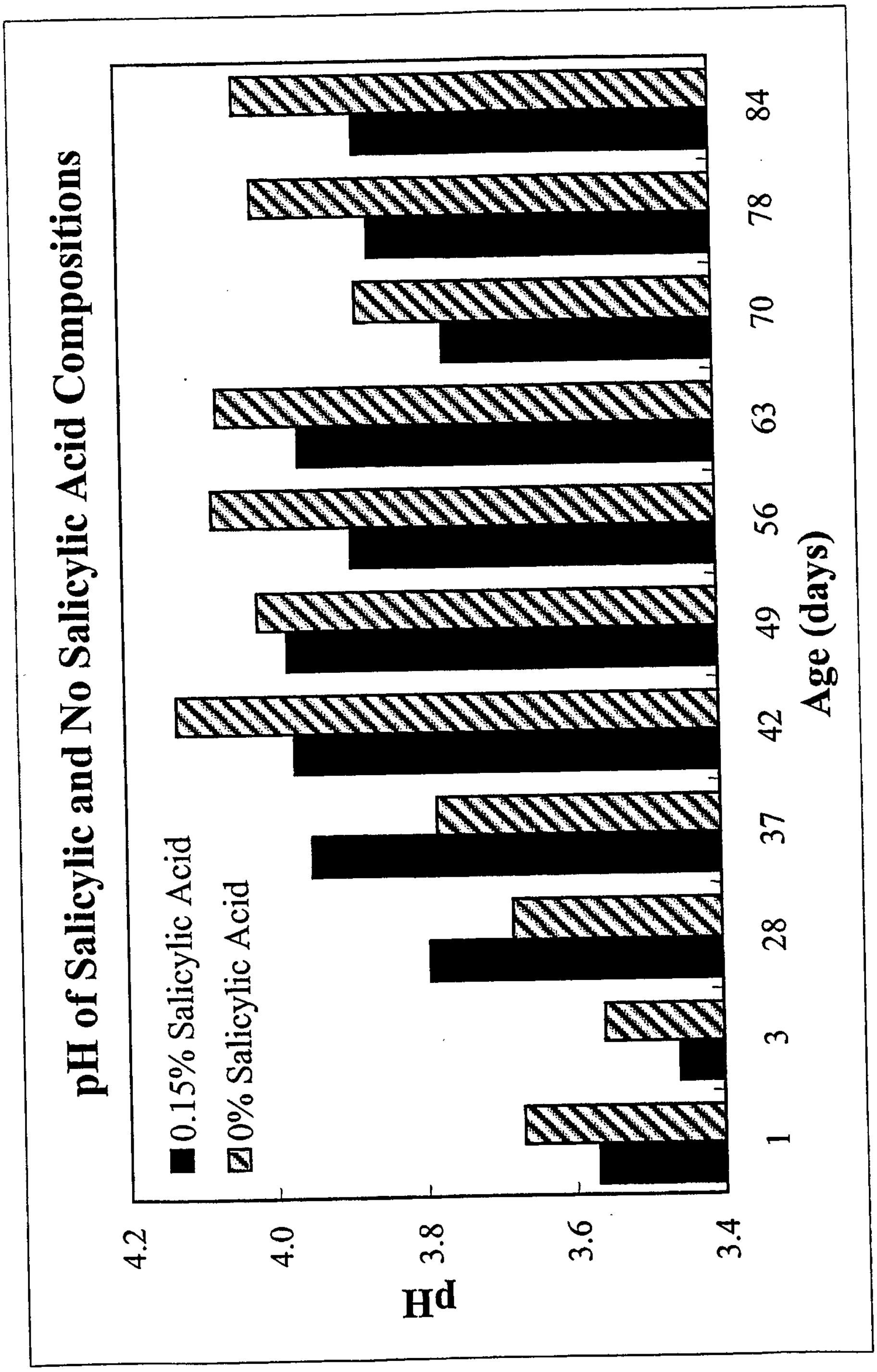
pH of Salicylic and No Salicylic Acid Compositions
0.15% Salicylic Acid
0% Salicylic Acid
pH
4.2
4.0
3.8
3.6
3.4
1
3
28
37
42
49
56
63
70
78
84
Age (days)

Figure 2

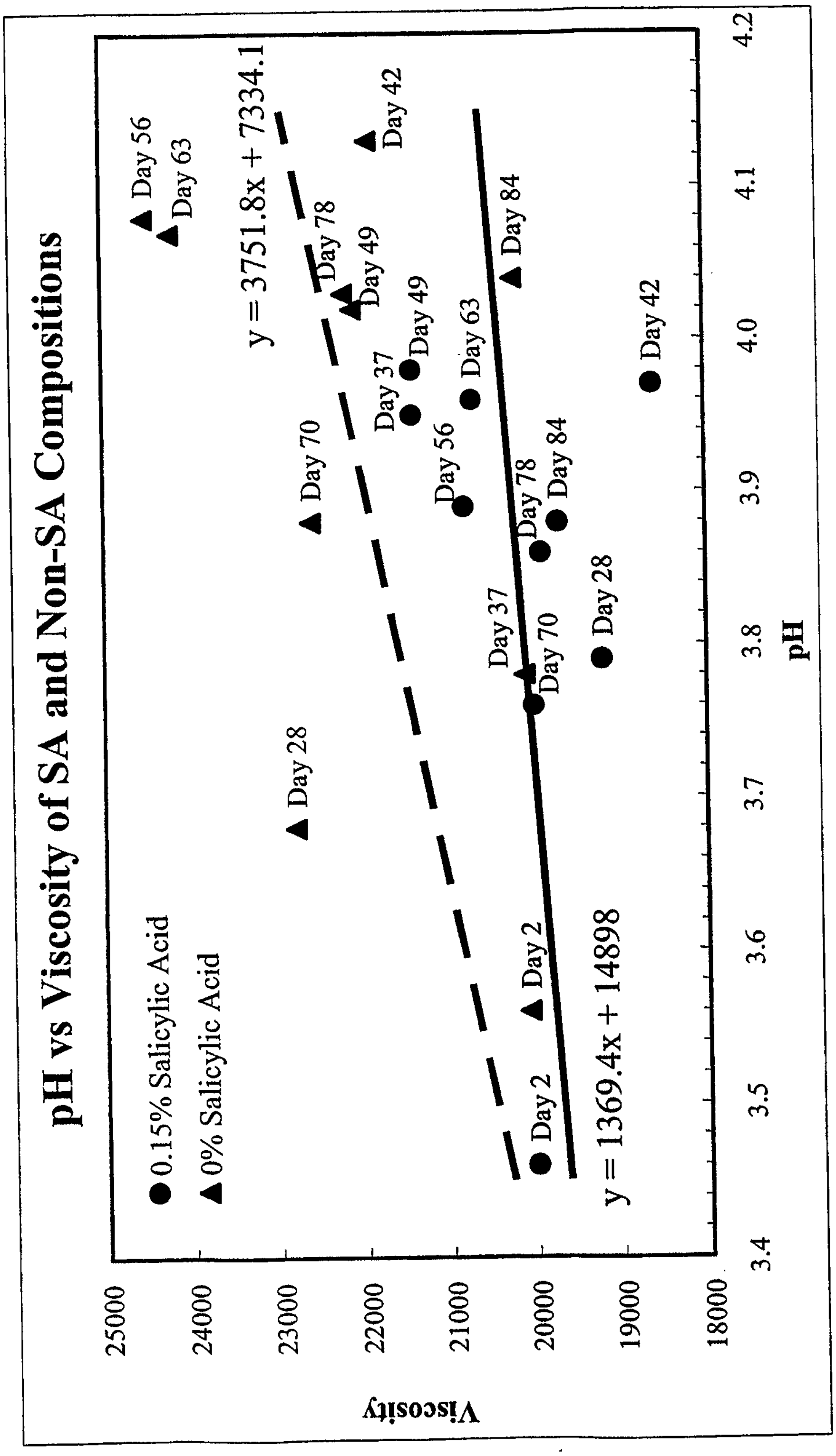
pH vs Viscosity of SA and Non-SA Compositions
0.15% Salicylic Acid
0% Salicylic Acid
y = 3751.8x + 7334.1
y = 1369.4x + 14898
Day 2
Day 2
Day 28
Day 28
Day 37
Day 37
Day 42
Day 42
Day 49
Day 49
Day 56
Day 56
Day 63
Day 63
Day 70
Day 70
Day 78
Day 78
Day 84
Day 84
Viscosity
25000
24000
23000
22000
21000
20000
19000
18000
pH
3.4
3.5
3.6
3.7
3.8
3.9
4.0
4.1
4.2

Figure 3

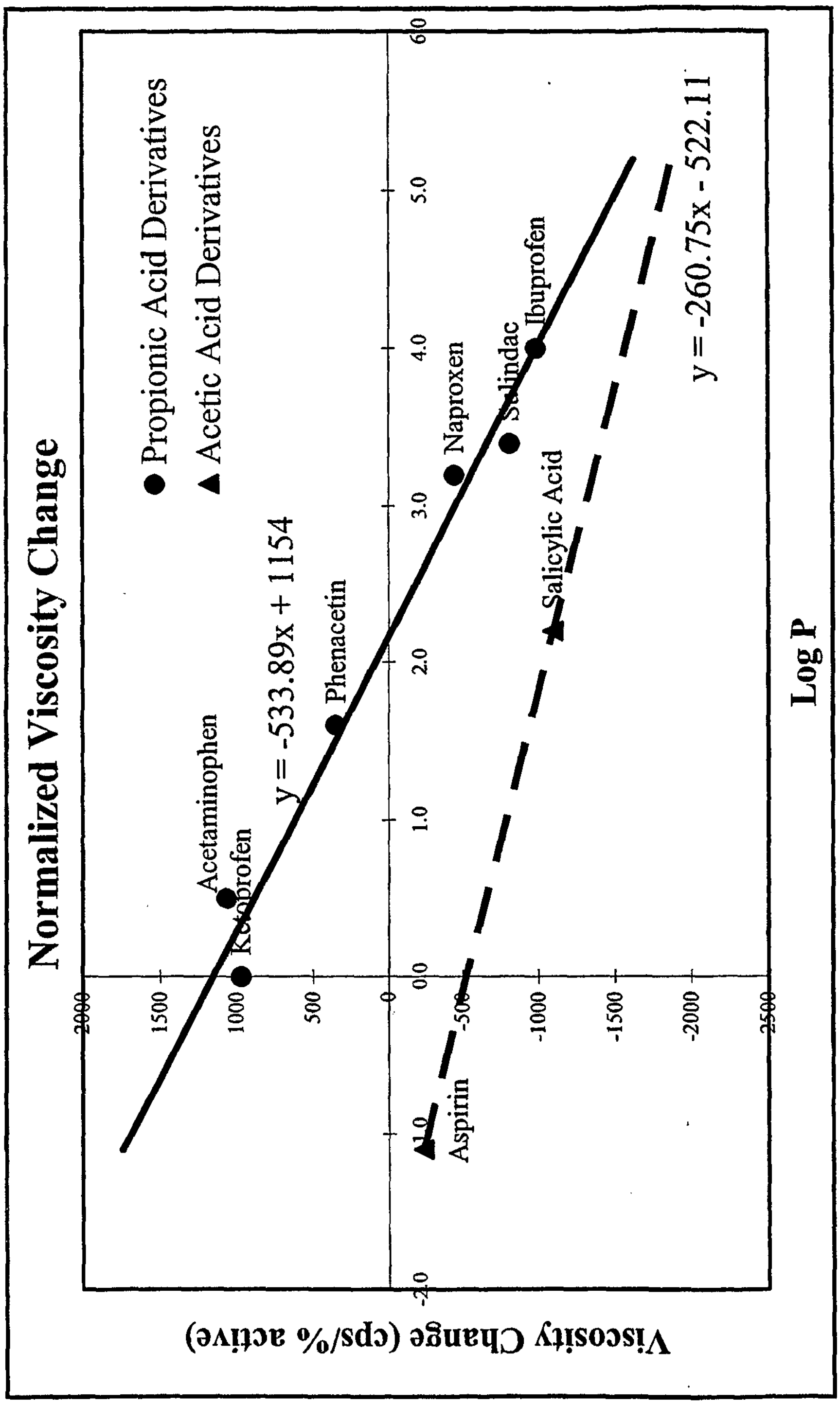
Normalized Viscosity Change
● Propionic Acid Derivatives
▲ Acetic Acid Derivatives
y = -533.89x + 1154
y = -260.75x - 522.11
Acetaminophen
Ketoprofen
Phenacetin
Naproxen
Sulindac
Ibuprofen
Salicylic Acid
Aspirin
Viscosity Change (cps/% active)
2000
1500
1000
500
0
-500
-1000
-1500
-2000
-2500
Log P
-2.0
-1.0
0.0
1.0
2.0
3.0
4.0
5.0
6.0

Figure 4

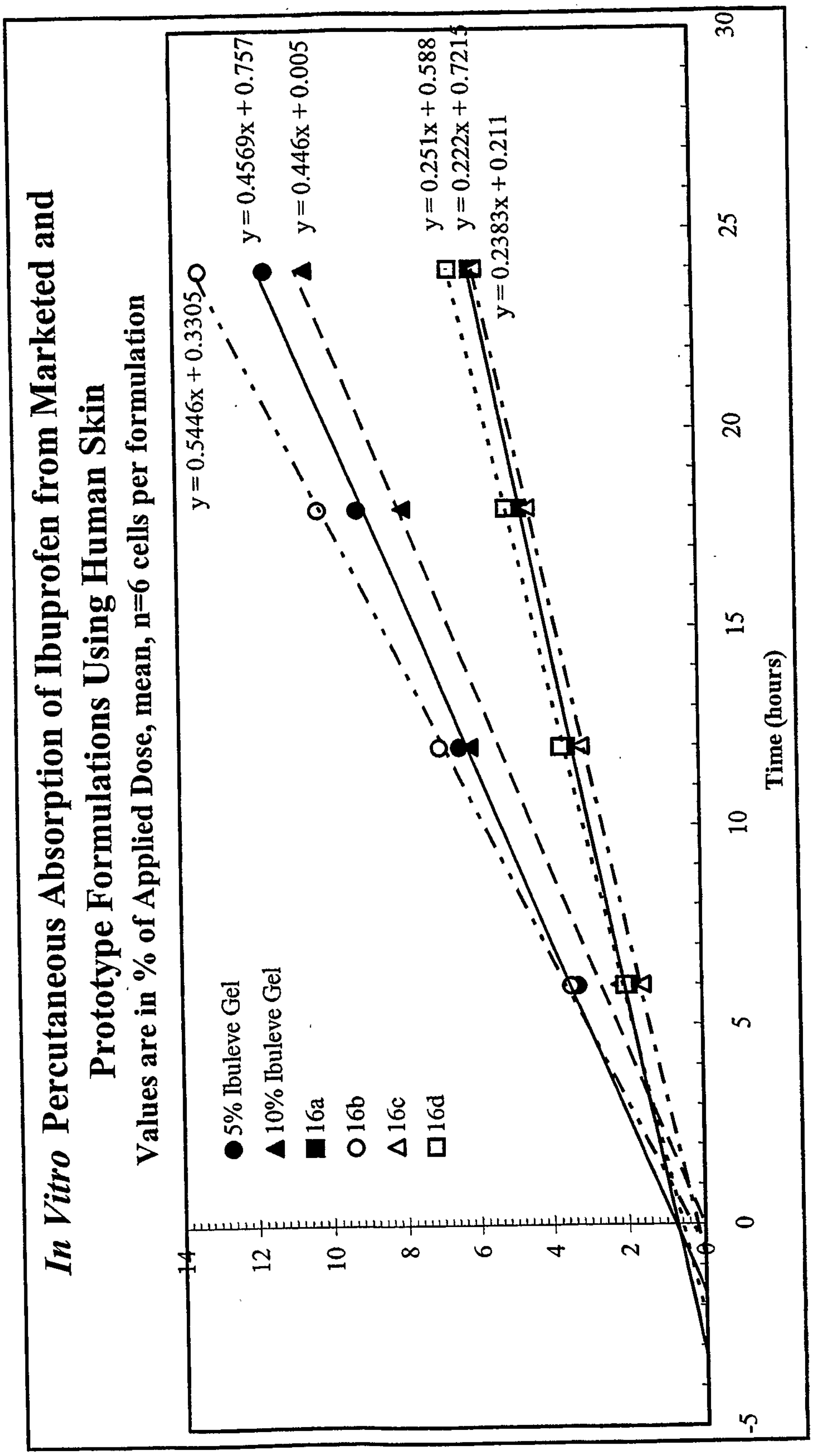
In Vitro Percutaneous Absorption of Ibuprofen from Marketed and Prototype Formulations Using Human Skin
Values are in % of Applied Dose, mean, n=6 cells per formulation
5% Ibuleve Gel
10% Ibuleve Gel
16a
16b
16c
16d
y = 0.5446x + 0.3305
y = 0.4569x + 0.757
y = 0.446x + 0.005
y = 0.251x + 0.588
y = 0.2222x + 0.7215
y = 0.2383x + 0.211
Time (hours)
-5
0
5
10
15
20
25
30
2
4
6
8
10
12
14

Figure 5

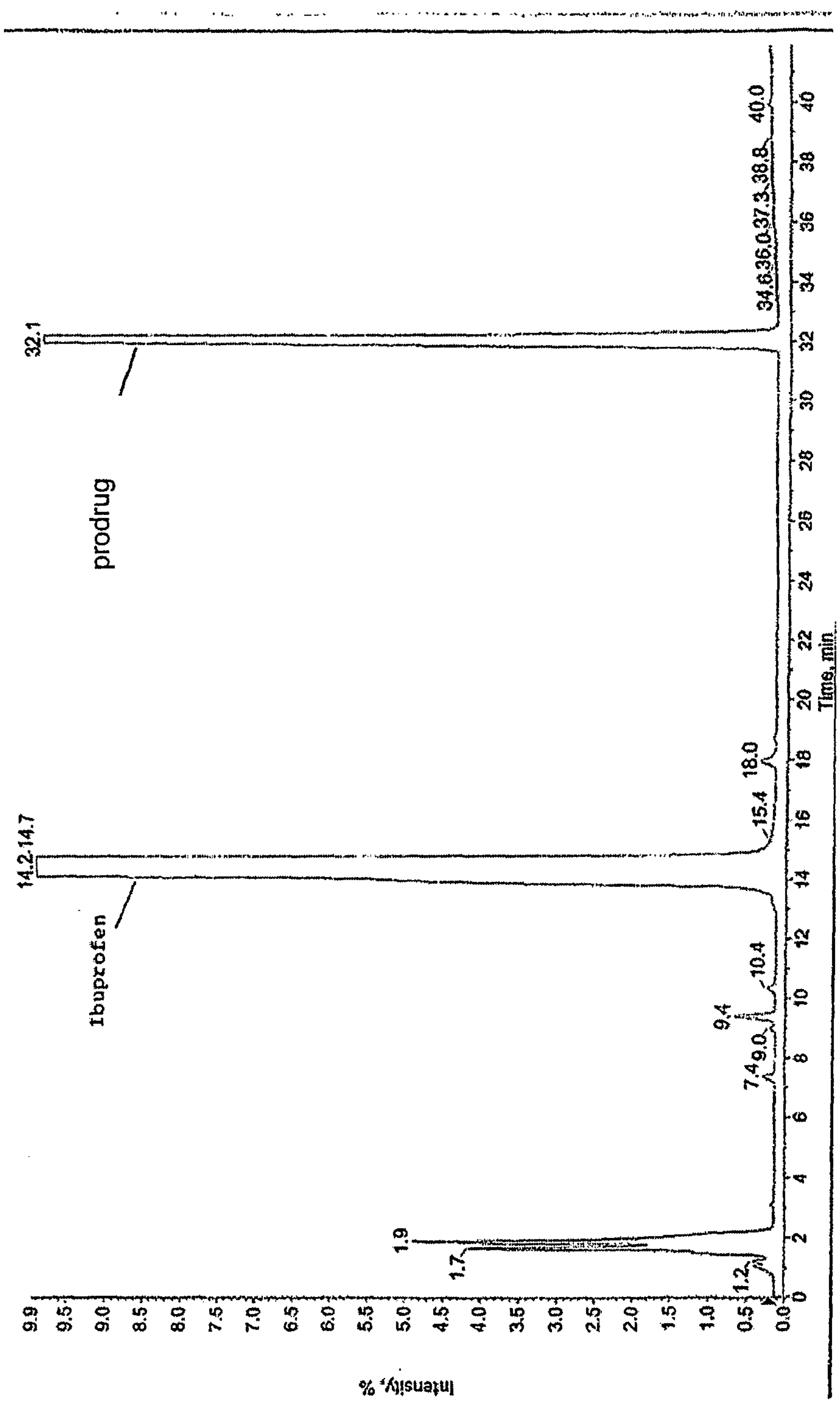
prodrug
Ibuprofen
32.1
14.2 14.7
1.9
1.7
1.2
7.4 9.0
9.4
10.4
15.4
18.0
34.6 36.0 37.3 38.8
40.0
Time, min
Intensity, %

Figure 6

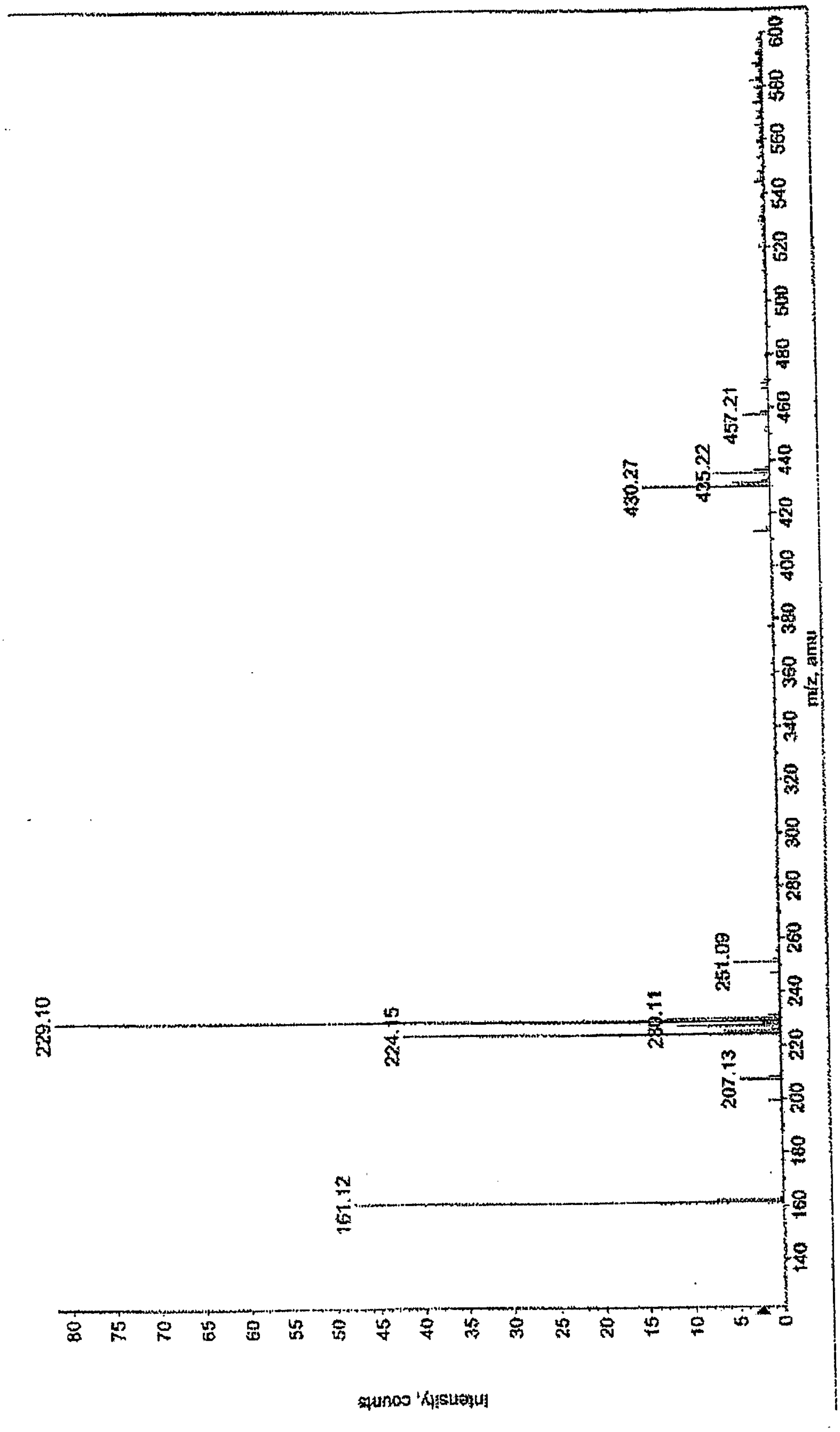

Intensity, counts
m/z, amu
229.10
224.15
161.12
207.13
251.09
430.27
435.22
457.21


Figure 7a

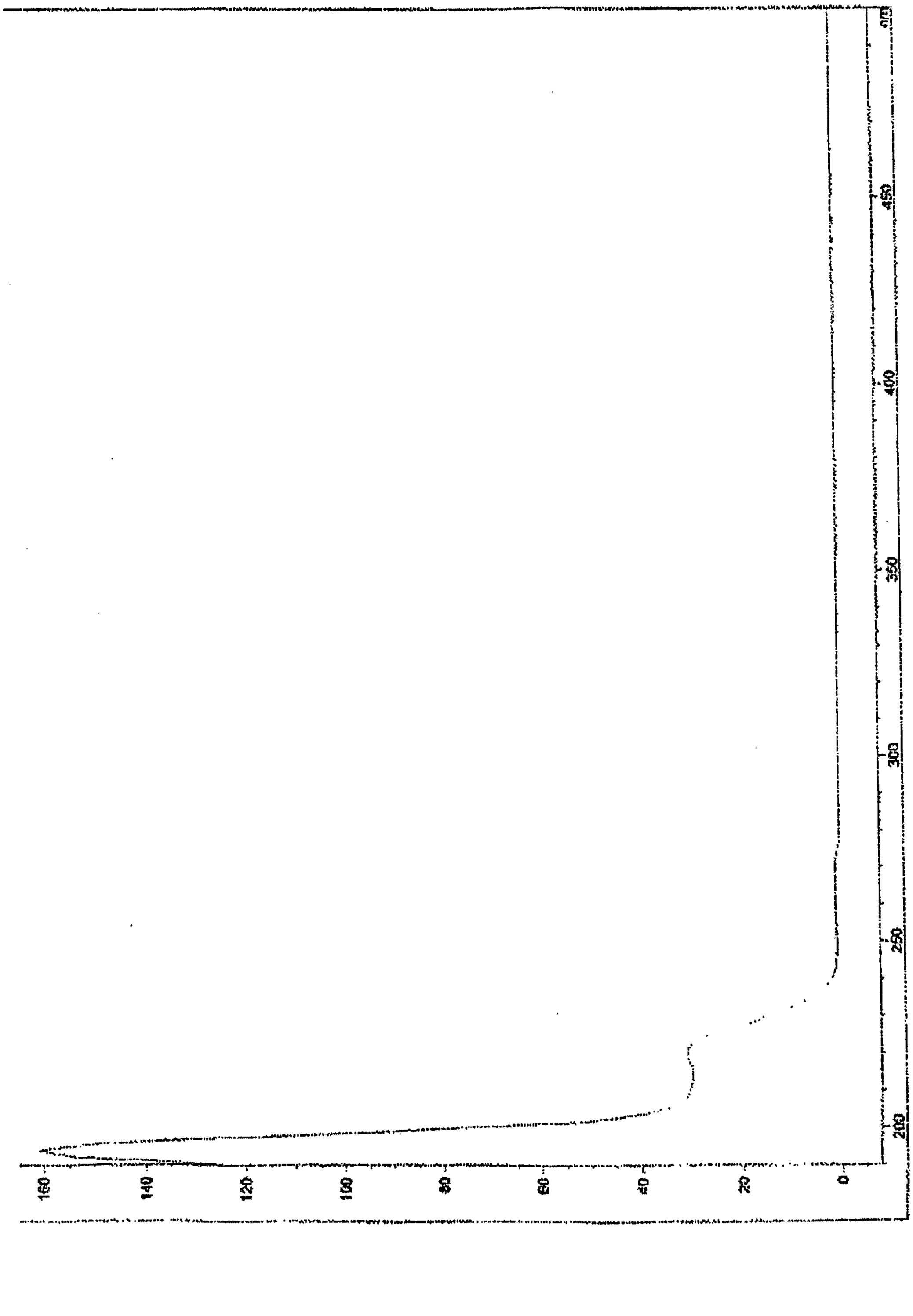


Figure 7b

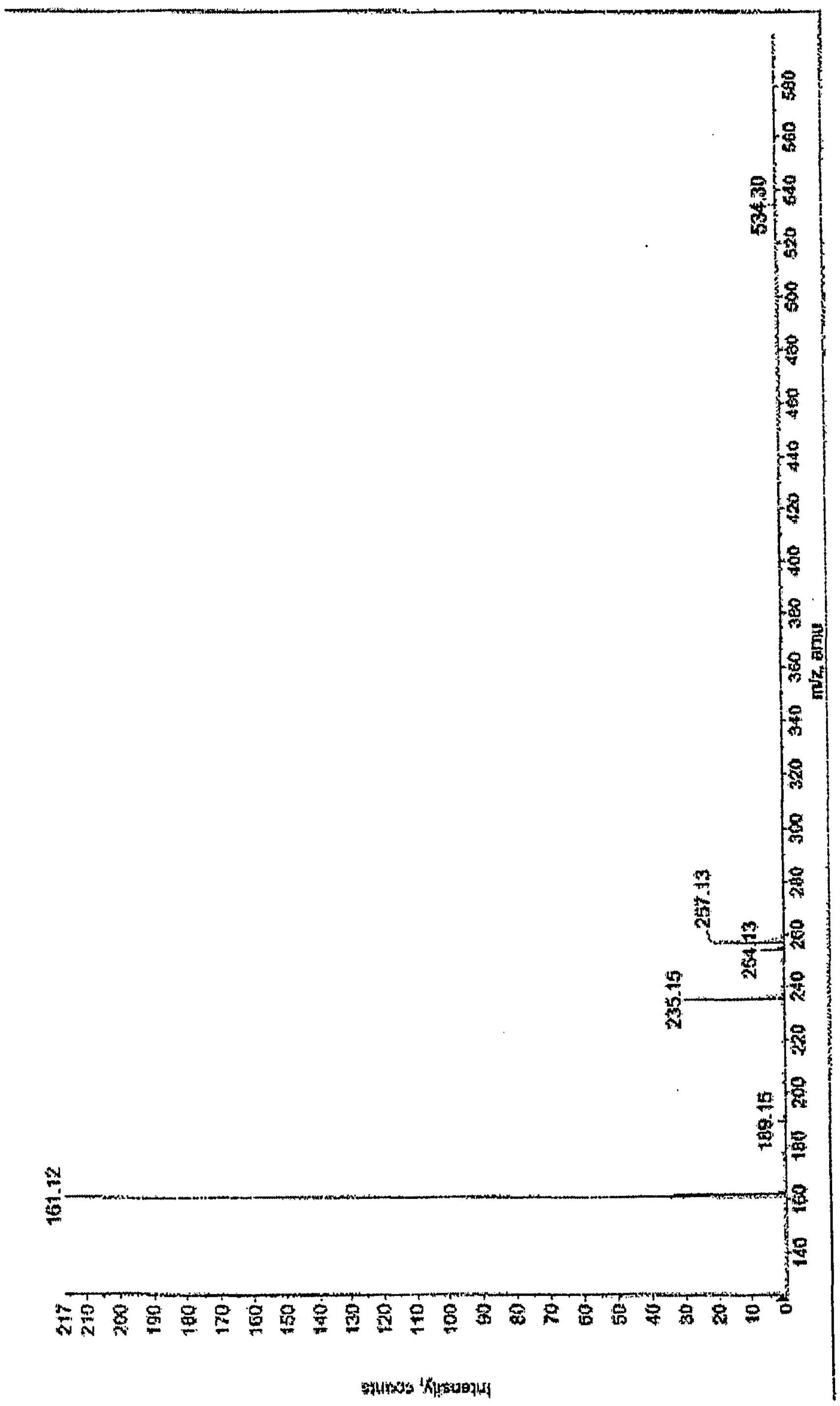
161.12
189.15
235.15
254.13
257.13
534.30
m/z, amu
Intensity, counts

Figure 8a

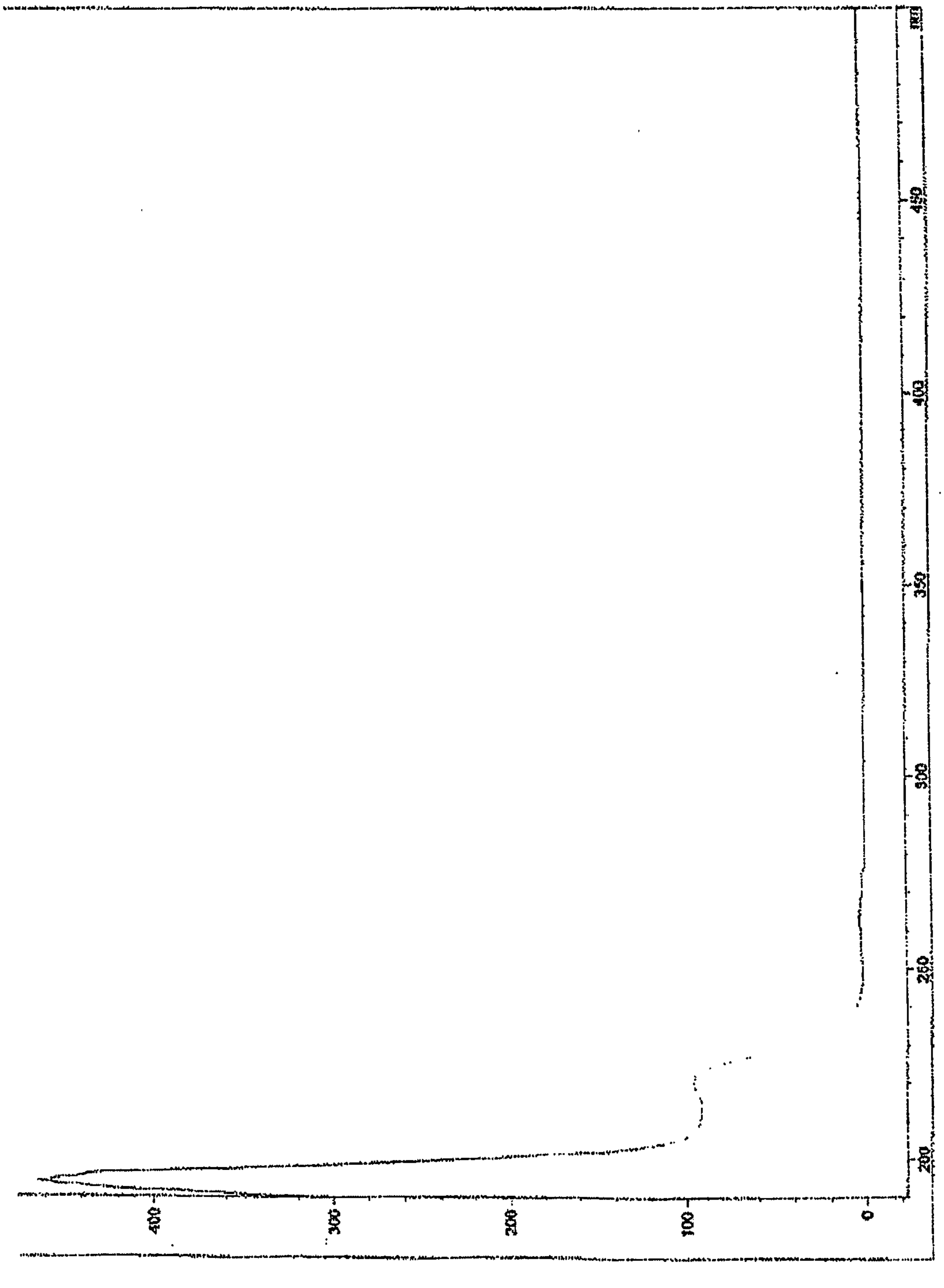


Figure 8b

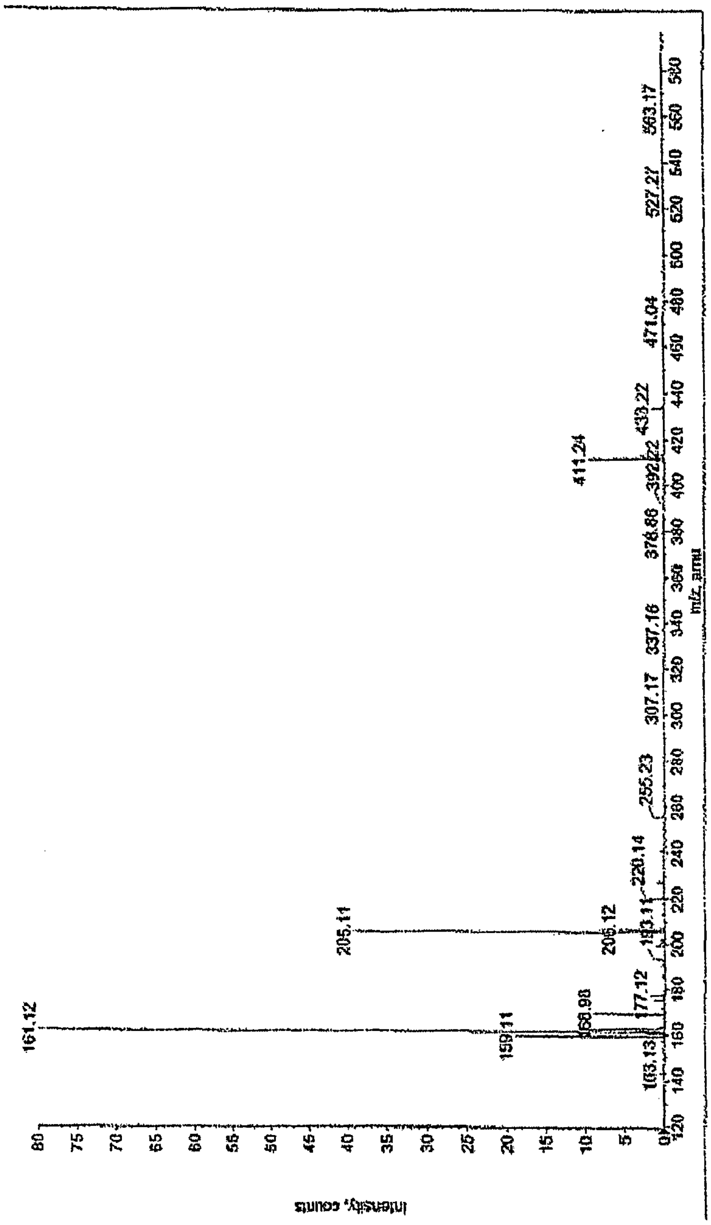
Intensity, counts
m/z, amu
161.12
205.11
159.11
163.13
168.98
177.12
193.11
206.12
220.14
255.23
307.17
337.16
376.86
392.22
411.24
433.22
471.04
527.27
563.17

Figure 9

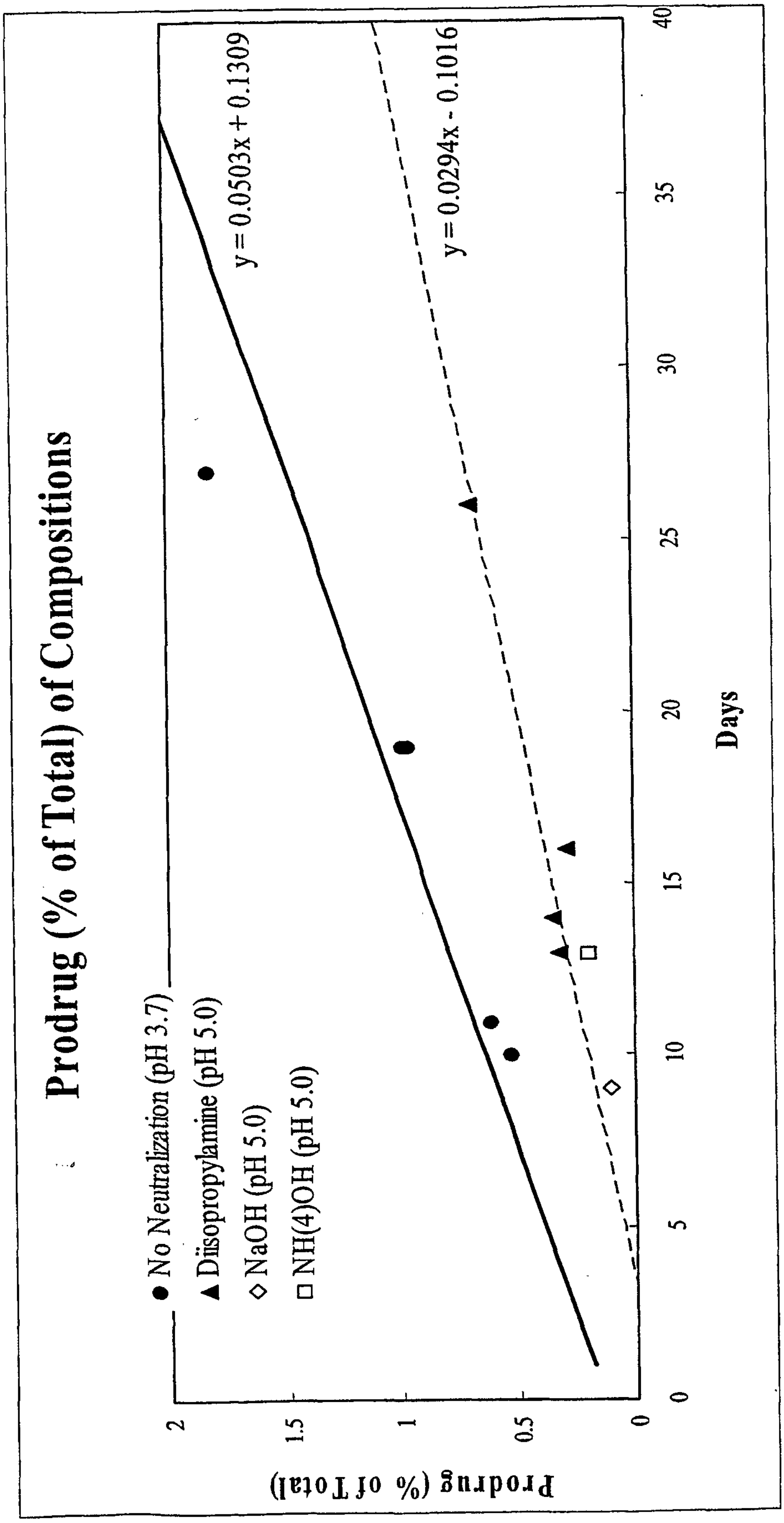
Prodrug (% of Total) of Compositions
● No Neutralization (pH 3.7)
▲ Diisopropylamine (pH 5.0)
◇ NaOH (pH 5.0)
□ NH(4)OH (pH 5.0)
y = 0.0503x + 0.1309
y = 0.0294x - 0.1016
Prodrug (% of Total)
0
0.5
1
1.5
2
Days
0
5
10
15
20
25
30
35
40

Figure 10

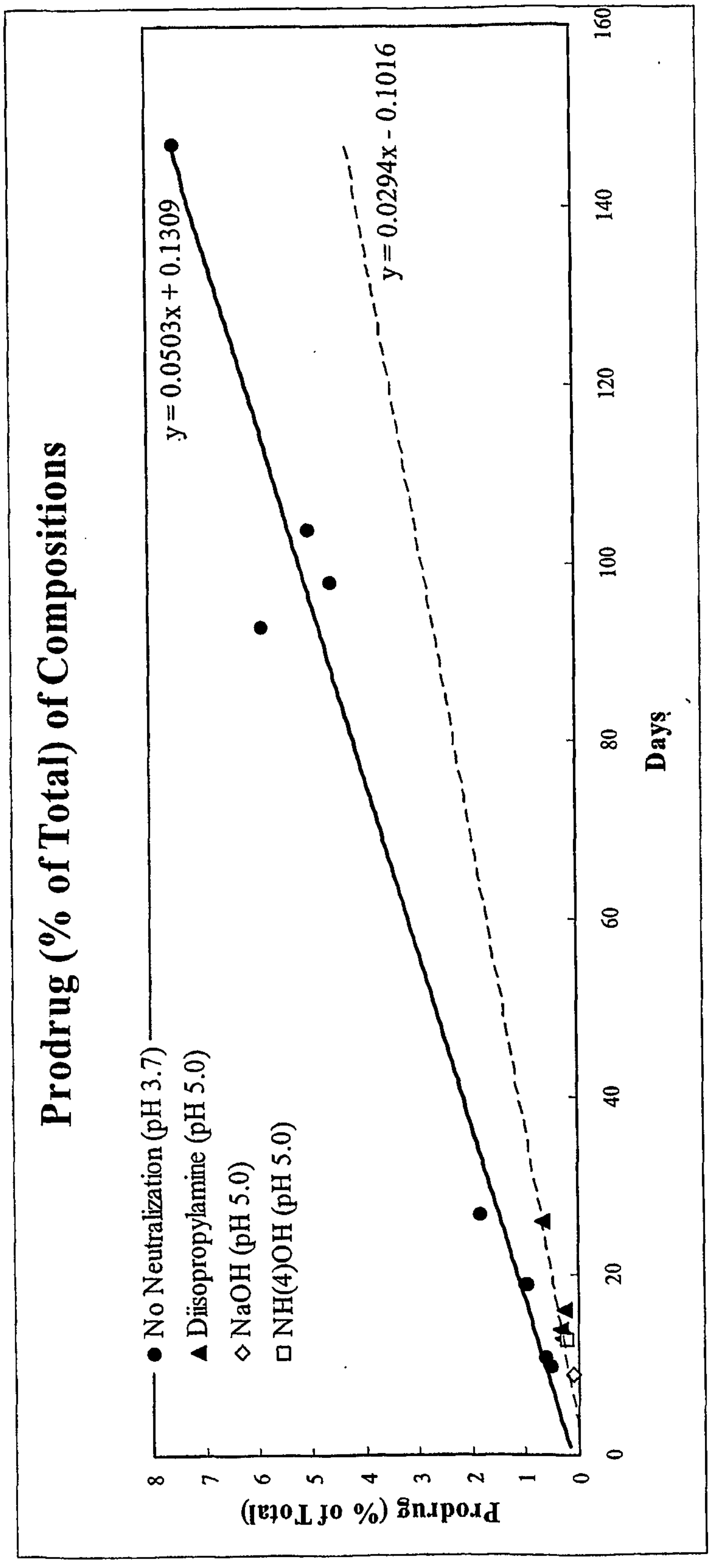
Prodrug (% of Total) of Compositions
No Neutralization (pH 3.7)
Diisopropylamine (pH 5.0)
NaOH (pH 5.0)
NH(4)OH (pH 5.0)
y = 0.0503x + 0.1309
y = 0.0294x - 0.1016
Prodrug (% of Total)
0
1
2
3
4
5
6
7
8
0
20
40
60
80
100
120
140
160
Days

Figure 11

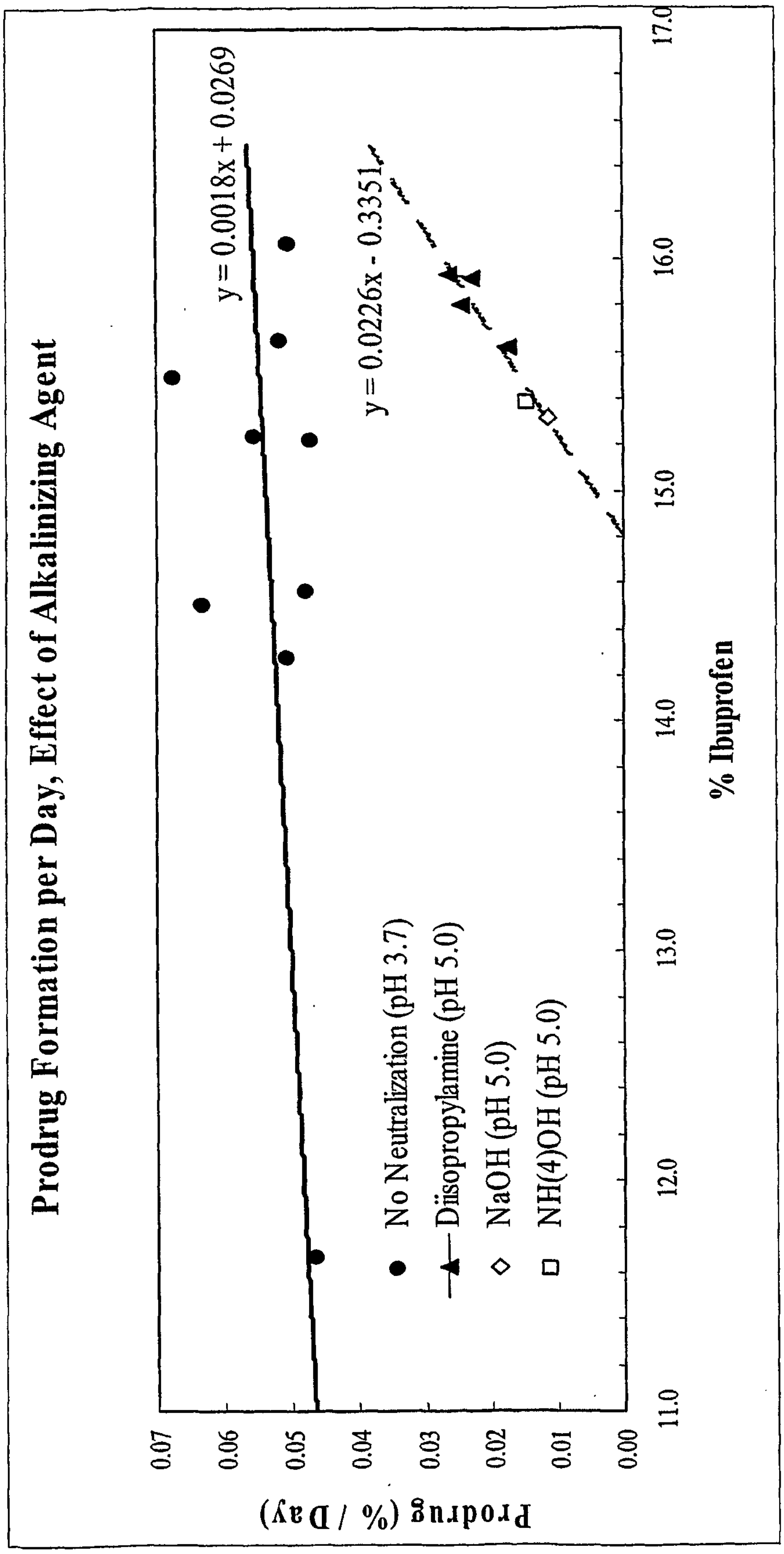
Prodrug Formation per Day, Effect of Alkalinizing Agent
y = 0.0018x + 0.0269
y = 0.0226x - 0.3351
No Neutralization (pH 3.7)
Diisopropylamine (pH 5.0)
NaOH (pH 5.0)
NH(4)OH (pH 5.0)
Prodrug (% / Day)
0.07
0.06
0.05
0.04
0.03
0.02
0.01
0.00
11.0
12.0
13.0
14.0
15.0
16.0
17.0
% Ibuprofen

Figure 12

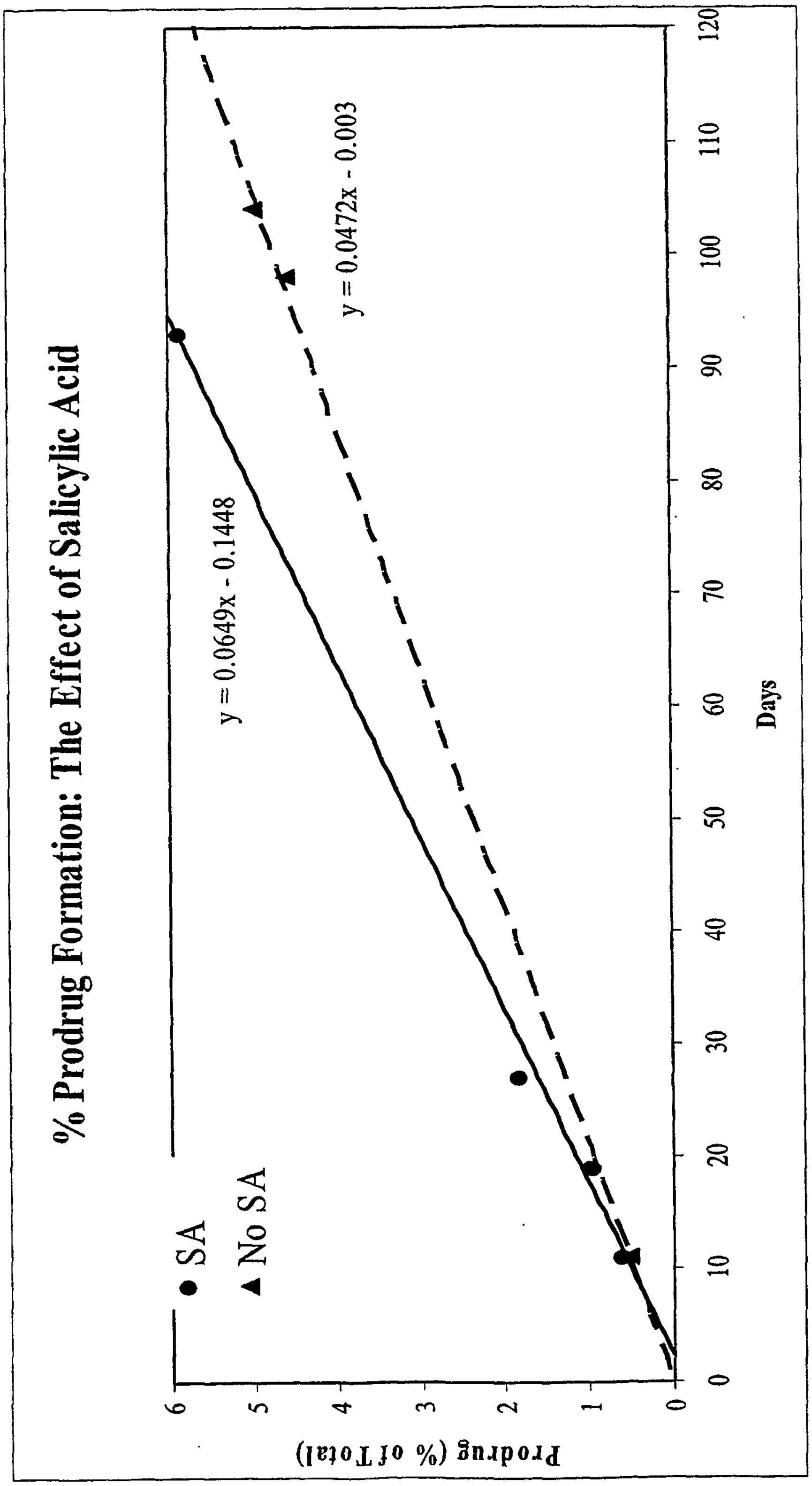
% Prodrug Formation: The Effect of Salicylic Acid
SA
No SA
y = 0.0649x - 0.1448
y = 0.0472x - 0.003
Prodrug (% of Total)
0
1
2
3
4
5
6
0
10
20
30
40
50
60
70
80
90
100
110
120
Days

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 65808405 P
- US 68110205 P
- US 69020105 P
- US 4185100 A
- EP 0151953 A
- US 5093133 A
- US 4533546 A
- US 6277362 B, Ita
- US 3981681 A, Mario de la Guarida
- US 4228163 A, William E. Bliss
- US 4525344 A, Ronald J. Tutsky
- US 4775530 A, Nicholas V. Perricone
- US 5034221 A, Steven E. Rosen
- US 5976566 A, Samour
- US 5914322 A
- EP 0439344 A2
- WO 8702891 A
- US 5073641 A
- US 5998465 A
- US 5811438 A
- US 6730696 B
- US 6620813 B
- US 6143734 A
- US 5750564 A
- US 5484833 A
- US 5315027 A
- US 4990658 A
- US 4851426 A
- US 4049700 A
- US 3228831 A


### Non-patent literature cited in the description


- **KYUKI et al.** Anti-Inflammatory Effect of Diclofenac-Sodium Ointment (Cream) in Topical Application. *Japan J. Pharmacol.,* 1983, vol. 33, 121-132
- **FRESNO et al.** *Eur. J. Pharm. Biopharm.,* 2002, vol. 54, 329-335
- **AKBARI et al.** *FIP World Congress Proceedings,* 2002
- **A.K. BANSAL et al.** *Indian Journal of Experimental Biology,* March 2001, vol. 39 (3), 280-283
- *Pharm. Res.,* vol. 4 (265), 87
- *J Dermatol.,* March 1992, vol. 19 (3), 140-5
- *Journal of Investigative Dermatology,* October 2001, vol. 117 (4), 977